# EUROPEAN PATENT SPECIFICATION

(11) **EP 3 702 459 B1**
(45) Date of publication and mention of the grant of the patent: **04.10.2023**
(21) Application number: 18870546.1
(22) Date of filing: 26.10.2018
(51) Int. Cl.: C12N 15/113, C12N 15/90, C12N 5/0789, A61P 7/06, A61K 35/12, A61K 35/28, A61K 48/00

(54) **METHOD FOR IMPROVING FETAL HEMOGLOBIN EXPRESSION**
VERFAHREN ZUR VERBESSERUNG DER EXPRESSION VON FETALEM HÄMOGLOBIN
PROCÉDÉ D'AMÉLIORATION DE L'EXPRESSION DE L'HÉMOGLOBINE FOETALE

(30) Priority: 27.10.2017 CN 201711027708
(43) Date of publication of application: 02.09.2020
(73) Proprietor: EdiGene (GuangZhou) Inc., Guangzhou City, Guangdong 510000 (CN)
(72) Inventor: YUAN, Pengfei, Beijing 102206 (CN); FANG, Riguo, Beijing 102206 (CN); YU, Lingling, Beijing 102206 (CN); XIA, Penghui, Beijing 102206 (CN); WANG, Jia, Beijing 102206 (CN); LIANG, Fucai, Beijing 102206 (CN)
(74) Representative: Lavoix
(86) International application number: PCT/CN2018/112027
(87) International publication number: WO 2019/080917

(56) References cited:
- WO-A1-2016/182917
- WO-A1-2017/115268
- WO-A2-2015/073683
- WO-A2-2017/182881
- WO-A2-2017/182881
- US-A1- 2016 369 262
- SHAH SIDDHARTH ET AL: "Concise Review: Stem Cell-Based Approaches to Red Blood Cell Production for Transfusion", STEM CELLS TRANSLATIONAL MEDICINE, vol. 3, no. 3, 20 December 2013 (2013-12-20), pages 346-355, XP055814031, US ISSN: 2157-6564, DOI: 10.5966/sctm.2013-0054
- Wang Yebo: "The Optimization of CRISPR/Cas9 and Its Application in Human Stem Cells", Doctoral Dissertation, no. 6, 15 June 2017 (2017-06-15), pages 1-134, XP009520648, ISSN: 1674-022X
- STUART H. ORKIN.: "Recent advances in globin research using genome-wide asso- ciation studies and gene editing", Annals of the New York Academy of Sciences, vol. 1368, no. 1, 31 March 2016 (2016-03-31), pages 5-10, XP055274553, ISSN: 0077-8923

## Description

### TECHNIQUE FIELD

The present invention relates to a cell treatment regimen for treating anemia diseases such as thalassemia, sickle cell anemia, and the like, and it comprises efficiently and safely performing in vitro genetic modification of a enhancer locus of BCL11A in human hematopoietic stem cells by gene editing technology, especially disrupting a BCL11A genomic region of the consecutive bases of CTTCCT in the chromosome 2 in the hematopoietic stem cells by in vitro gene editing technology, and up-regulating the expression of γ-globin and fetal hemoglobin, thereby achieving the purpose of treating diseases.

### BACKGROUND OF THE INVENTION

Thalassemia, also known as globin aplastic anemia or mediterranean anemia, is a group of hemolytic anemia diseases caused by genetic factors such as genetic defects. Hereditary genetic defects result in the loss or lack of synthesis of one or more globin peptide chains in hemoglobin, leading to a pathological state of anemia or hemolysis. The reduction or loss of the globin chains for synthesizing hemoglobin leads to abnormal structure of hemoglobin. The deformability of an erythrocyte containing abnormal hemoglobin is reduced, and its life span is shortened. *In situ* hemolysis may occur in bone marrow, and when entering the peripheral blood circulation, the erythrocytes will be destroyed in advance by organs such as spleen, thereby resulting in anemia, iron deposition in the body and even abnormal development. Due to the diversity and complexity of gene mutations for different globin chains, the types, quantity of the deficient globin and the clinical symptoms show apparent variation.

Thalassemia is named and classified according to the types of the deficient globin chains and the deficient degree thereof. According to different types of globin peptide chain formation disorders, thalassemia may be divided into α type, β type, δ type, and δβ type. At present, thalassemia is one of the most common genetic diseases with gene defects in the world. According to statistics, 4.83% of the global population carries globin mutant genes, including 1.67% of α, β thalassemia heterozygotes, and 1.92% of them carrying hemoglobin with a sickle cell mutation, 0.95% carrying hemoglobin E, and 0.29% carrying hemoglobin C, etc. It is a common gene-deficient disease, resulting the global birth rate of the population with abnormal hemoglobin and disease symptoms is no less than 0.024%.

β thalassemia is a type of thalassemia, and its pathogenesis is due to the gene mutation in the β globin peptide chain, for most patients it is a point mutation, while for a few patients it is a large segment deletion of the gene. Gene deletions and certain point mutations result in complete inhibition of synthesis of a part of the β globin peptide chain, and this kind of condition is called β⁰ thalassemia. While a few point mutations partly inhibit the synthesis of the β chain, but still retain the synthesis of some part of the peptide chain, and this type of condition is called β⁺ thalassemia. Different combinations of mutations may cause different clinical symptoms. There are many gene mutation types of β thalassemia. According to statistics, there are more than 300 genetic abnormalities around the world, more than 100 mutation sites have been found, and currently 28 mutation sites have been clinically reported in China. Among them, there are 6 common mutations, including about 45% of β41-42 (-TCTT deletion), about 24% of IVS-II654 (C to T point mutation), about 14 % of β17 (A to T point mutation), about 9% of TATA box-28 (A to T point mutation), about 2% of B71-72 (+A insertion mutation), and about 2% of β26 (G to A point mutation), etc.

There are two mutant gene types for severe β thalassemia, one is the β homozygote, and the other is double heterozygote of β⁰ and β⁺ thalassemia. Since the formation of β globin peptide chain is nearly completely inhibited, and the β globin chain cannot be produced in vivo, the normal synthesis of hemoglobin A consisting of α chain and β chain is reduced or eliminated. Although the excess α chain proteins can bind to the γ chains in an erythrocyte to form hemoglobin F, as the synthesis of γ chain is gradually inhibited (also known as hemoglobin chain synthesis switching) after birth, the excess α chain is deposited in erythrocytes to form inclusion bodies adhering to the surface of erythrocyte membrane. Such that the characteristics of erythrocyte membrane are changed, the cells become stiff to cause a decrease in deformability, and they are destroyed in the bone marrow to result in "ineffective hematopoiesis". Although some erythrocytes of a thalassemia patient can develop and mature in the bone marrow, and eventually be released into the peripheral blood circulation, when they pass through the peripheral local microcirculation (such as the spleen and other organs), mechanical breakage will be easily occurred due to the decrease of their deformability. For the above reasons, a baby patient is clinically asymptomatic at birth. After birth, due to the expression of HbF (fetal hemoglobin) and the up to 120 days life span of erythrocytes, the chronic hemolytic anemia is often shown 6 months later when the cell damage is increased by its pathological changes, i.e., γ chain synthesis is physiologically inhibited; hemoglobin chain synthesis is switched to β chain, and meanwhile β chain synthesis fails due to gene defects, and these further lead to changes in bone marrow composition. Repeated blood transfusions are required in the treatment process, resulting in hemosiderosis and thereby affecting the functions of important organs.

Similar to β thalassemia, sickle-shaped erythrocyte anemia is an autosomal recessive genetic disease, except that this anemia has a single mutation site. It is caused by the single base mutation of β globin, i.e., the codon 6 for the normal β gene is mutated from GAG (encoding glutamic acid) to GTG (encoding valine). In the case of a mutant homozygote, normal α globin and abnormal β globin form a tetrameric complex called HbS, its capability of carrying oxygen is half of a normal hemoglobin, and it aggregates into multimers in the deoxygenated state. Since the resulting multimers are aligned parallel to the membrane, and closely contact with the cell membrane. When the number of multimers reaches a certain level, the cell membrane changes from a normal concave shape to a sickle shape. With poor deformability, the sickle-shaped erythrocytes break easily, and hemolysis is occurred, resulting in blood vascular blockage, injury, necrosis, and the like.

The treatments of β thalassemia and sickle cell anemia include: general supportive care, high-dose transfusions and regular iron chelation therapy, hematopoietic stem cell transplantation, drug therapy for inducing fetal hemoglobin, and exploratory gene therapy. At present, allogeneic hematopoietic stem cell transplantation, among all the treatments, is the only hopeful therapy for cure. Since the first hematopoietic stem cell transplantation in a thalassemia patient was successfully carried out in 1981 by Thomas, the hematopoietic stem cell transplantation technology has been used in several thalassemia research centers around the world, and it successfully replaced the traditional blood transfusion and iron chelation treatment regimen. However, the widespread use of hematopoietic stem cell transplantation in the treatment of thalassemia is still limited, due to the severe shortage of HLA-matched donors and the death caused by GVHD (graft-versus-host disease) after transplantation. At the same time, researchers have been continuously exploring drugs for the treatment of thalassemia. At present, the only oral drug approved by FDA for clinical treatment is hydroxyurea, which alleviates the clinical symptoms of the disease mainly by inducing the expression of fetal hemoglobin. However, the clinical efficacy of this drug is inconsistent and there are significant large side effects, as well as dose-related myelosuppression. It is urgent to develop new therapeutic methods for the treatment of the anemia related diseases such as β thalassemia, and sickle cell anemia.

WO 2017/115268, WO 2016/182917 and WO 2017/182881 relate to sgRNAs targeting BCL11A in the region from chr2:260495236-chr2:260495271. WO 2017/115268 discloses sgRNAs comprising a nucleotide sequence of SEQ: 3, 4, or 8, wherein the sgRNAs comprise 2'O-methyl and phosphorothioate modifications.

### SUMMARY

According to the present disclosure, a new generation of hematopoietic stem cells are developed by gene editing technology, such as CRISPR/Cas9 gene editing technology. Compared with the hematopoietic stem cells in the prior art, the knockout efficiency of BCL11A enhancer gene in the hematopoietic stem cells is significantly increased, thereby greatly improving the expression of fetal hemoglobin in erythrocytes after differentiation and maturation, and in a certain extent solving the problem in the prior art that the editing efficiency of BCL11A enhancer is low and the expression of fetal hemoglobin cannot satisfy the clinical application. In addition, the present invention further improves the strategy for culturing and differentiating the hematopoietic stem cells subjected to gene editing, not only greatly shortening the differentiation process from hematopoietic stem cells to mature erythrocytes, but also greatly increasing the number of the harvested mature erythrocytes, and thereby partially satisfying the requirements for clinical application.

In particular, the invention relates to the subject-matter according to the appended claims.

### Advantages

With experiments the present invention proves that, through disrupting a BCL11A genomic region from chr2:60495236 to chr2:60495271 (e.g., the 6-bp base sequence of CTTCCT) in the BCL11A gene, the inhibition of fetal hemoglobin synthesis by the BCL11A gene may be eliminated to increase the expression of fetal hemoglobin, indicating that the "CTTCCT" sequence and its adjacent regions are key regions for regulating the expression of fetal hemoglobin by the BCL11A gene.

Herein, gene-edited hematopoietic stem cells are prepared by disrupting a BCL11A genomic region of CTTCCT (chr2:60495251-60495256) in the chromosome 2 in the hematopoietic stem cells by gene editing technology, and it is proved by experiments that erythroid differentiation of said gene-edited hematopoietic stem cells can significantly increase the expression of γ globin and fetal hemoglobin (HbF); and the hematopoietic system of an animal model can be reconstituted by infusion them into the animal. At the same time, off-target analysis shows that the method is highly safe, and almost no side effect caused by gene editing is detected.

### BRIEF DESCRIPTION OF THE DRAWINGS

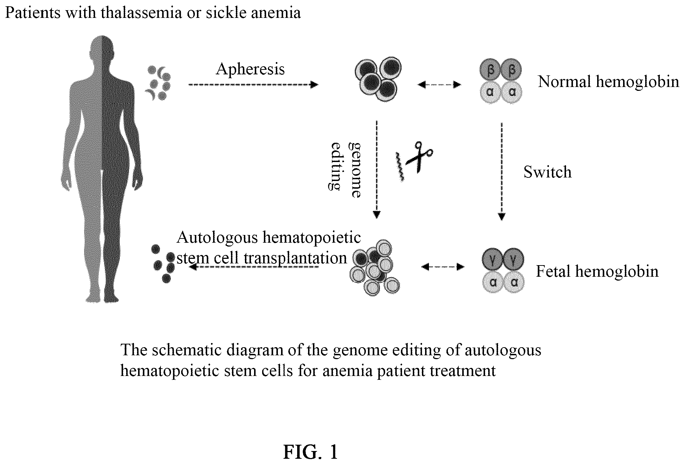

**FIG. 1****:** The therapeutic schedule for the treatment of β thalassemia and sickle cell anemia with genetically modified autologous hematopoietic stem cells. Peripheral blood is mobilized from a patient, CD34-positive hematopoietic stem cells are obtained by in vitro isolation and genetically edited in vitro to improve the expression of fetal hemoglobin, and then the genetically modified autologous hematopoietic stem cells are returned to the patient.

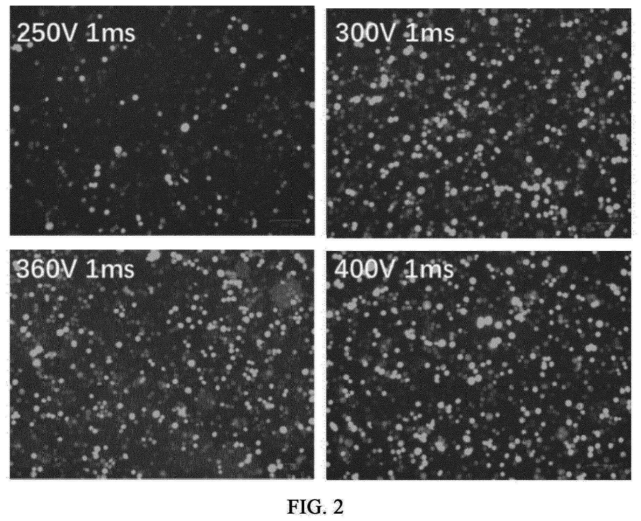

**FIG. 2****:** Fluorescence photomicrographs, obtained 4 days after transfecting with *GFP* (*green* fluorescent protein) by electroporation in a multi-batch assay on the cancer cell line K562 for determining the optimal electroporation conditions. "V" refers to the pulse voltage and "ms" refers to the pulse time.

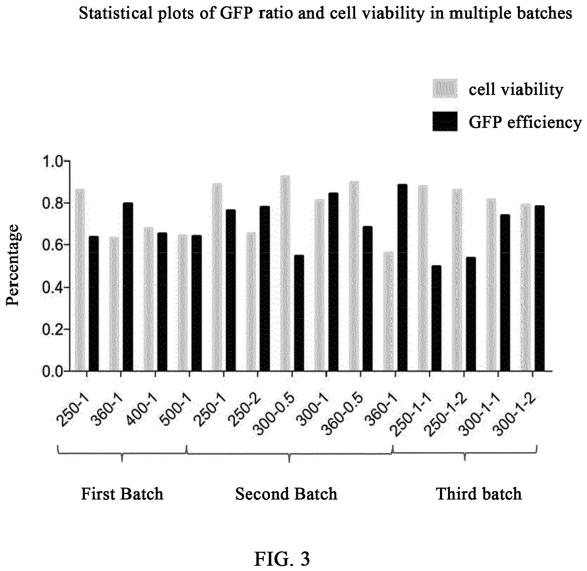

**FIG. 3****:** A graph showing flow cytometry analysis of 7-AAD and statistical analysis of GFP expression, obtained 4 days after transfecting with *GFP* by electroporation in a multi-batch assay on the cancer cell line K562 for determining the optimal electroporation conditions. 7-AAD indicates the viability of cells. 7-AAD (7-amino-actinomycin D) is a nucleic acid dye that does not pass through the normal plasma membrane. During the process of apoptosis and cell death, the permeability of the plasma membrane to 7-AAD is gradually increased; being excited by excitation light with appropriate wavelength, 7-AAD emits bright red fluorescence, and normal viable cells are negative for 7-AAD; GFP refers to electroporation efficiency. "250-1" refers to a voltage of 250V, and a pulse time of 1ms; "250-1-1", "250-1-2" respectively indicate the two repetitions of "250V, 1ms"; "300-1-1", "300-1-2" respectively indicate the two repetitions of "300V, 1ms".

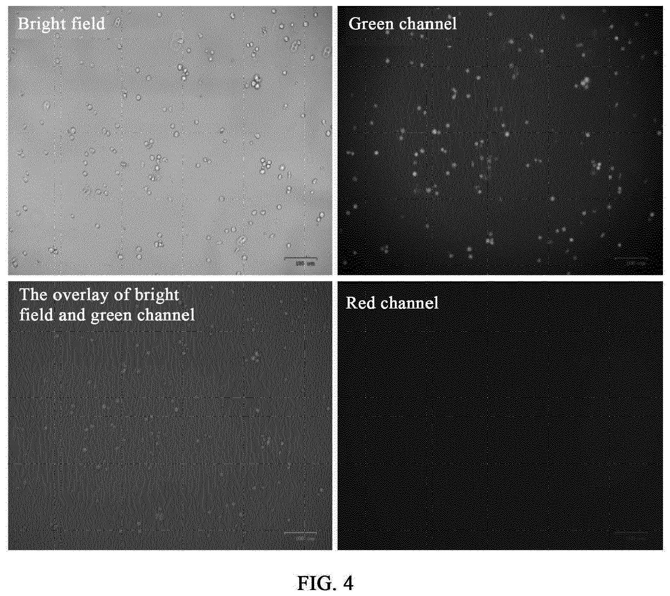

**FIG. 4****:** Fluorescence photomicrographs, obtained 4 days after transfecting the hematopoietic stem cells with GFP mRNA by electroporation under the electroporation conditions of "300V, 1ms". The four fields included are the bright field, green channel, red channel, and overlay of the bright field and green channel.

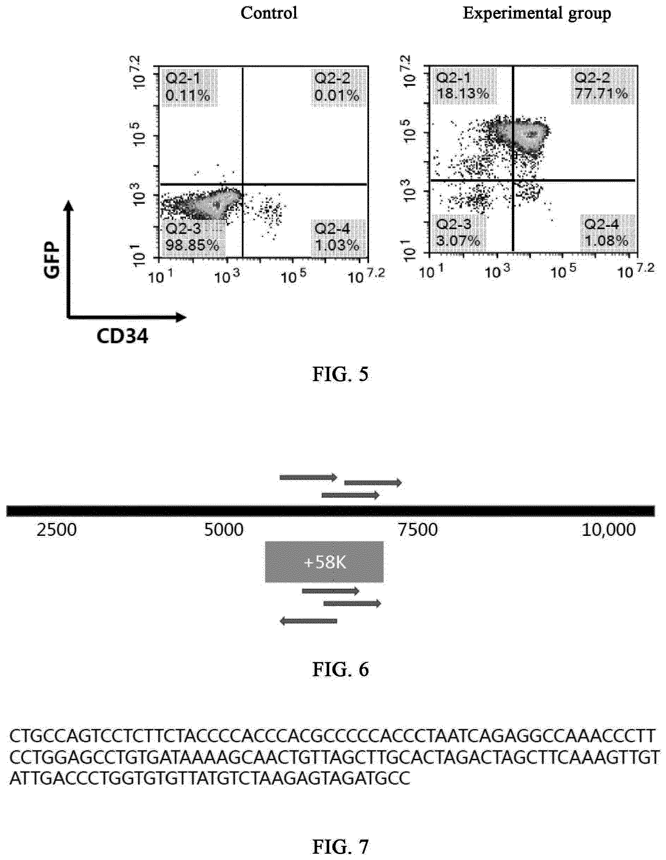

**FIG. 5****:** Flow cytometry analysis of GFP and expression of CD34 protein, obtained 4 days after transfecting the hematopoietic stem cells with GFP mRNA by electroporation under the electroporation conditions of "300V, 1ms".
**FIG. 6****:** A schematic diagram of multiple sgRNAs designed for the locus 58K of human BCL11A enhancer.
**FIG. 7****:** Information of the 150-bp sequence at the locus 58K of human BCL11A enhancer.

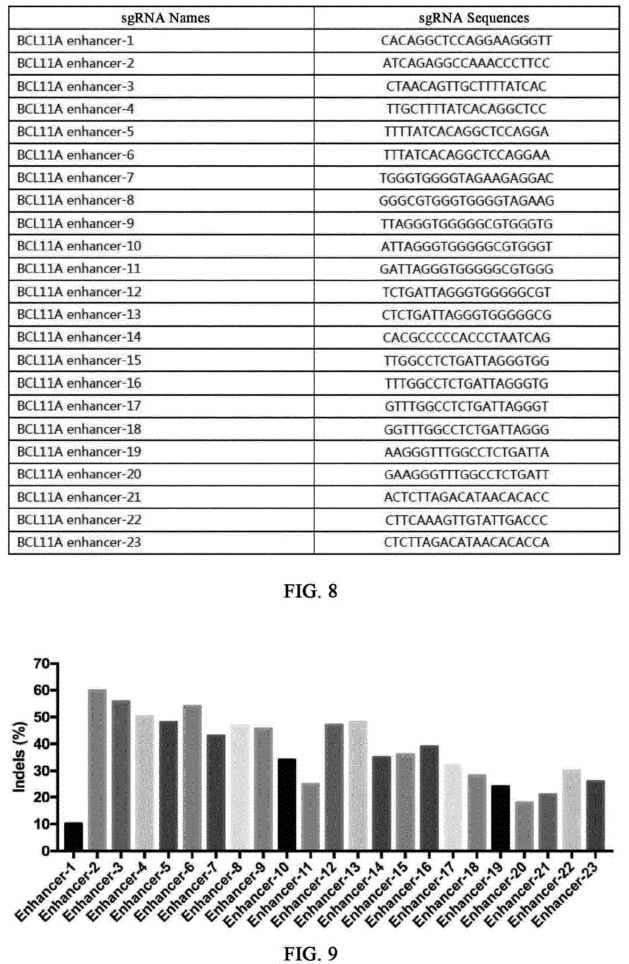

**FIG. 8****:** Specific DNA sequence information of 23 sgRNAs designed for the locus 58K locus (150 bp) of human BCL11A enhancer.
**FIG. 9****:** A graph showing the statistical analysis of Indels efficiency, which is obtained by transfecting CD34-positive hematopoietic stem cells with Cas9 mRNA and multiple sgRNAs by electroporation, then extracting the genomes 4 days later, amplifying the fragments and performing Sanger sequencing, and finally making said statistical analysis by TIDE software.

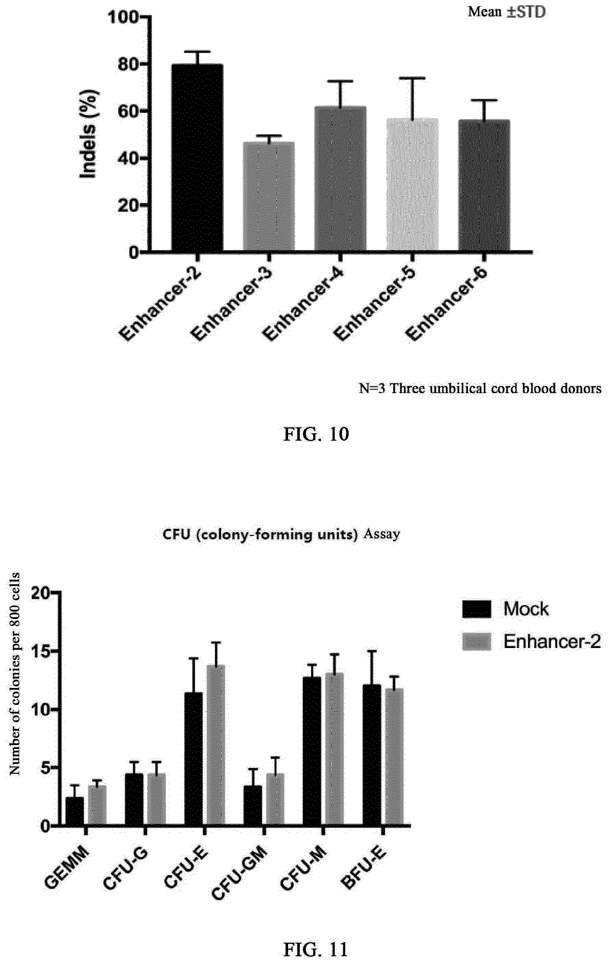

**FIG. 10****:** A graph showing the statistical analysis of Indels efficiency, which is obtained by transfecting CD34-positive hematopoietic stem cells derived from 3 different umbilical cord blood sources with Cas9 mRNA and BCL11A enhancer-2, 3, 4, 5, and 6 (i.e., enhancer-2, 3, 4, 5, and 6 in Fig. 10) sgRNAs by electroporation, then performing said statistical analysis by TIDE software after 4 days.
**FIG. 11****:** A graph showing the number of colonies for different blood systems, which is obtained by transfecting CD34-positive hematopoietic stem cells derived from umbilical cord blood with Cas9 mRNA and BCL11A enhancer-2 sgRNA by electroporation, performing in vitro colony-forming units assay (CFU assay) 2 days later, and then counting the number of colonies for different blood systems 14 days later; wherein BFU-E, CFU-M, CFU-GM, CFU-E, CFU-G, CFU-MM represent the cell colonies of different lineages such as the erythroid, myeloid, and lymphoid lineage, etc. in blood system, and wherein Mock represents cells not being genetically edited.

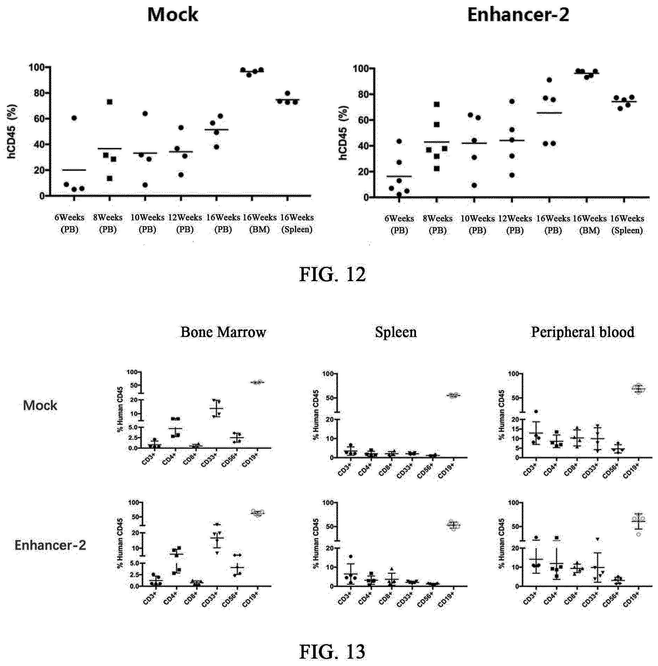

**FIG. 12****:** Graphs showing the proportion of human CD45-positive cells, which are obtained by transfecting the hematopoietic stem cells with Cas9 mRNA and BCL11A enhancer-2 sgRNA by electroporation, and transplanting the genetically modified and unmodified cells into NGF immune-deficient mouse model irradiated by irradiator respectively, after 6 weeks, 8 weeks, 10 weeks, 12 weeks, and 16 weeks, the proportion of human CD45-positive cells is detected in mouse peripheral blood; and 16 weeks after transplantation, the proportion of human CD45-positive cells is also detected in mouse bone marrow and spleen; wherein the proportion of CD45-positive cells is calculated through dividing human CD45-positive cell percentage (%) by (human CD45-positive cell percentage (%) + mouse CD45-positive cell percentage (%)); the human CD45-positive cell percentage (%) and mouse CD45-positive cell percentage (%) are respectively determined by flow cytometry assay; and wherein Mock represents cells not being genetically edited, and Enhancer-2 represents genetically modified cells.
**FIG. 13****:** Graphs showing the proportion of human CD45-positive cells, which are obtained by transfecting the hematopoietic stem cells derived from umbilical cord blood with Cas9 mRNA and BCL11A enhancer-2 sgRNA by electroporation, and transplanting the genetically modified and unmodified cells into NGF immune-deficient mouse model irradiated by irradiator respectively, 16 weeks later the proportion of human cell membrane proteins such as CD3, CD4, CD8, CD33, CD56, and CD19 versus human CD45 protein are respectively detected in mouse bone marrow, spleen, and peripheral blood; wherein Mock represents cells not being genetically edited, and Enhancer-2 represents genetically modified cells.

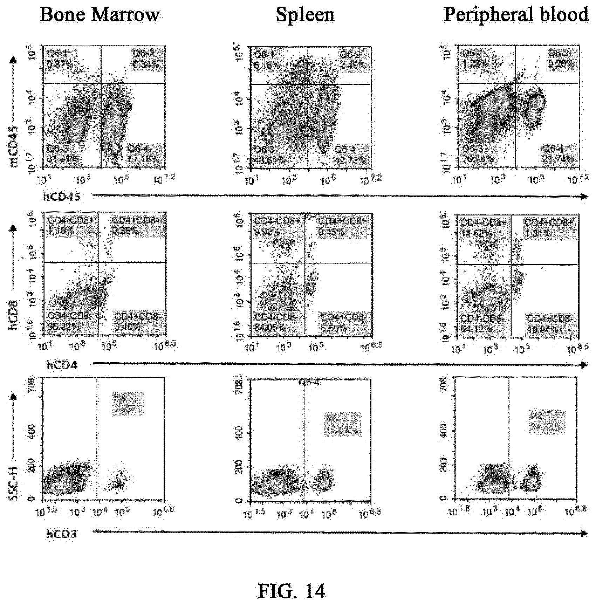

**FIG. 14****:** Graphs showing the expression of mouse CD45, human CD45, CD3, CD4 and CD8, which are obtained by transfecting the hematopoietic stem cells derived from umbilical cord blood with Cas9 mRNA and BCL11A enhancer-2 sgRNA by electroporation, and transplanting the genetically modified cells into NGF immune-deficient mouse model irradiated by irradiator, 16 weeks later the expressions of mouse CD45, human CD45, CD3, CD4 and CD8 are respectively detected in mouse bone marrow, spleen, and peripheral blood by flow cytometry assay; wherein the SSC-H channel refers to lateral angle scattering, which represents the granularity of the cells, and the larger value indicates larger granularity of the cells; the granularity refers to the degree of buckling on the cell surface, the number of subcellular organelles, and particles in a cell, etc.

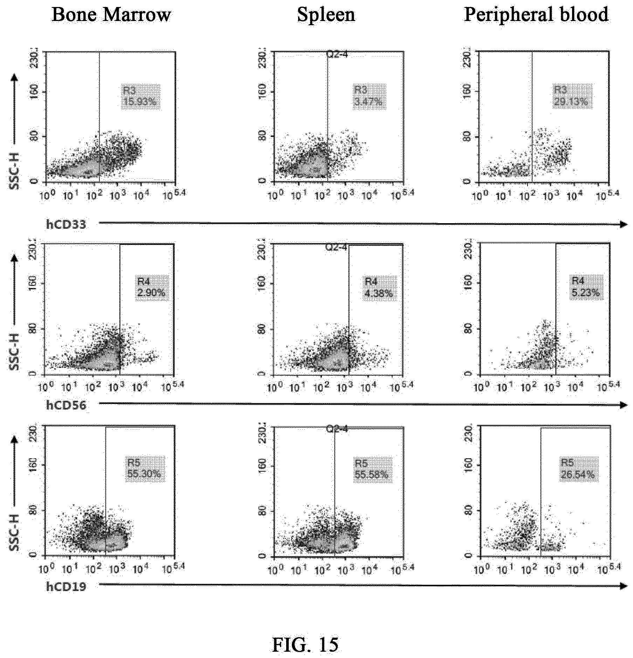

**FIG. 15****:** Graphs showing the expression of human CD33, CD56, and CD19 in one mouse, which are obtained by transfecting the hematopoietic stem cells derived from umbilical cord blood with Cas9 mRNA and BCL11A enhancer-2 sgRNA by electroporation, and transplanting the genetically modified cells into NGF immune-deficient mouse model irradiated by irradiator, 16 weeks later the expressions of human CD33, CD56, and CD19 are respectively detected in mouse bone marrow, spleen, and peripheral blood by flow cytometry assay; wherein the SSC-H channel refers to lateral angle scattering, which represents the granularity of the cells, and the larger value indicates larger granularity of the cells; the granularity refers to the degree of buckling on the cell surface, the number of subcellular organelles, and particles in a cell, etc.

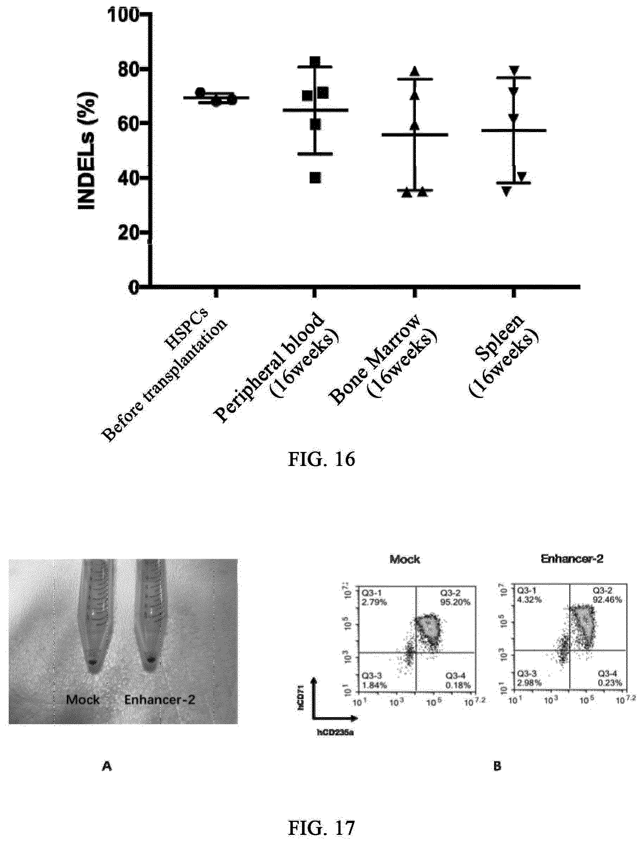

**FIG. 16****:** A graph showing statistical analysis of Indels efficiency, which is obtained by transfecting the hematopoietic stem cells derived from umbilical cord blood with Cas9 mRNA and BCL11A enhancer-2 sgRNA by electroporation, then extracting the genomes of the cells before transplantation, and genomes of peripheral blood, bone marrow, and spleen 16 weeks after transplantation, amplifying the fragments of the interest and performing Sanger sequencing, and finally analyzing Indels efficiency of gene editing by TIDE software.
**FIG. 17****:** Graphs showing erythroid differentiation, which are obtained by transfecting the hematopoietic stem cells derived from umbilical cord blood with Cas9 mRNA and BCL11A enhancer-2 sgRNA by electroporation, then performing erythroid differentiation, and detecting the cells after 12 days. Panel A is a photograph of the erythrocytes after differentiation; Panel B shows the detection results of the expression of two membrane proteins (human CD71 and human 235a), indicating the efficiency of erythroid differentiation; wherein Mock represents cells not being genetically edited, and Enhancer-2 represents genetically modified cells.

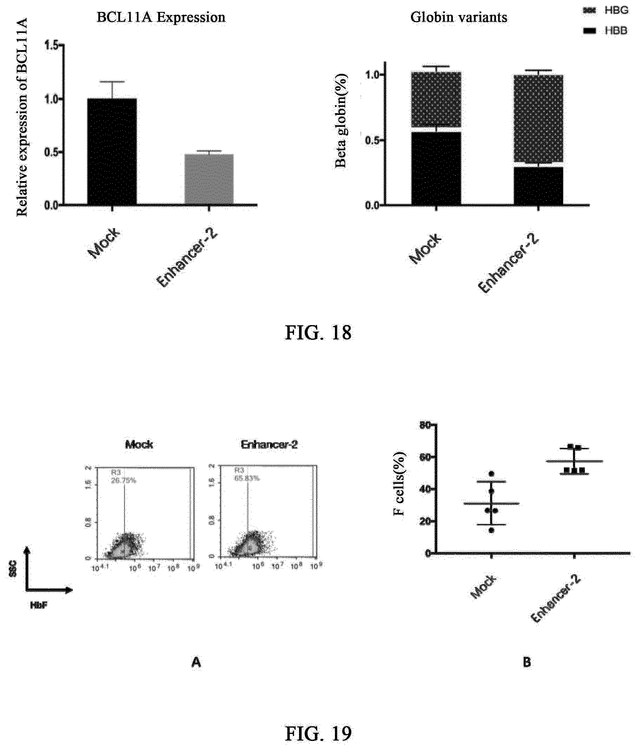

**FIG. 18****:** Graphs showing the expression of mRNAs, which are obtained by transfecting the hematopoietic stem cells derived from umbilical cord blood with Cas9 mRNA and BCL11A enhancer-2 sgRNA by electroporation, then performing erythroid differentiation, and detecting mRNA expression of BCL11A, HBB, HBG and other genes by quantitative fluorescence PCR 12 days later; wherein Mock represents cells not being genetically modified, and Enhancer-2 represents genetically modified cells.
**FIG. 19****:** Graphs showing the expression of fetal hemoglobin (HbF), which are obtained by transfecting the hematopoietic stem cells derived from umbilical cord blood with 6 µg of Cas9 mRNA and 4 ug of BCL11A enhancer-2 sgRNA by electroporation, then performing erythroid differentiation, and detecting the expression of fetal hemoglobin (HbF) in the cells after 12 days. The left panel shows the graphs about flow cytometry analysis, and the right panel is the statistical analysis graph about the expression of fetal hemoglobin; wherein Mock represents cells not being genetically modified, and Enhancer-2 represents genetically modified cells.

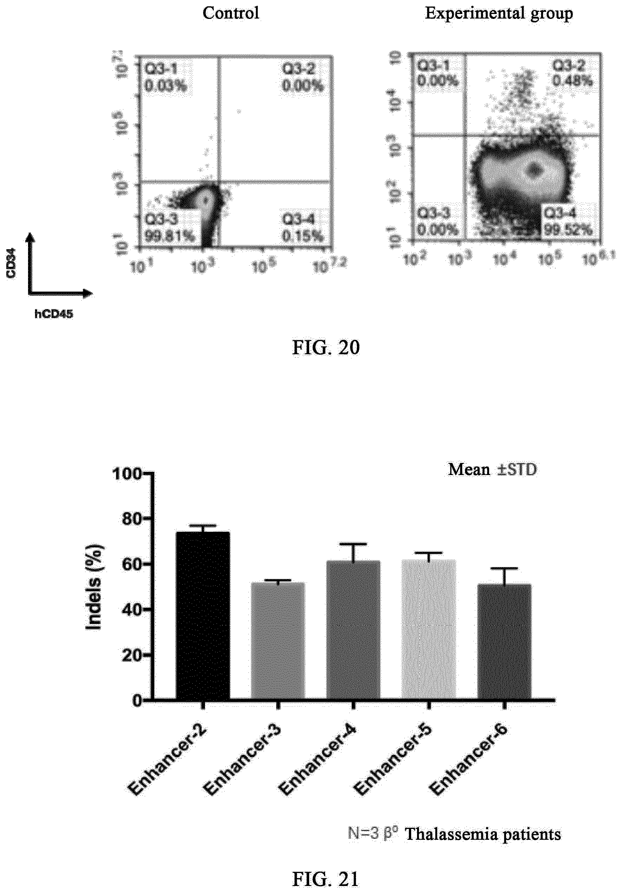

**FIG. 20****:** Graphs showing the detection results of the expression of CD45 and CD34 in freshly isolated peripheral blood of patients with β thalassemia. The left panel is the control group, and the right panel is the experimental group. The experimental samples are from 3 patients with β thalassemia respectively.
**FIG. 21****:** A graph showing the statistical analysis of Indels efficiency, which is obtained by transfecting CD34-positive hematopoietic stem cells isolated from peripheral blood of 3 different patients suffering from β thalassemia with Cas9 mRNA and BCL11A enhancer-2, 3, 4, 5, and 6 (i.e., enhancer-2, 3, 4, 5, and 6 in Fig. 21) sgRNAs by electroporation, after 4 days, performing statistical analysis of Indels efficiency by TIDE software.

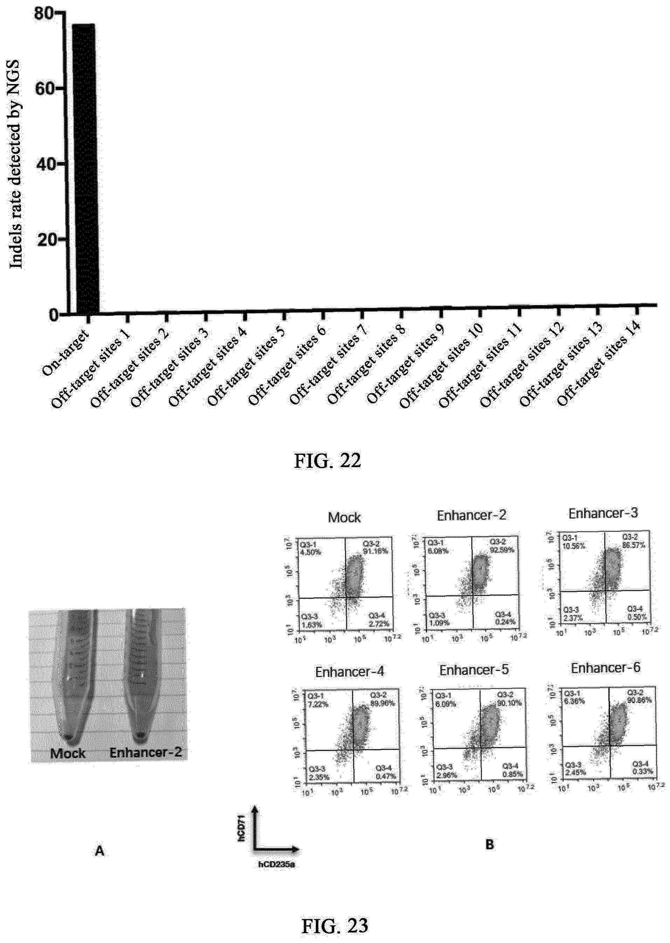

**FIG. 22****:** A graph showing the statistical analysis of potential off-target sites, which is obtained by transfecting CD34-positive hematopoietic stem cells isolated from peripheral blood of patients suffering from β thalassemia with Cas9 mRNA and BCL11A enhancer-2 sgRNA by electroporation, then extracting the genomes, and performing NGS deep sequencing analysis about the statistical graph of the 14 potential off-target sites.
**FIG. 23****:** Graphs showing the erythroid differentiation, which are obtained by transfecting the hematopoietic stem cells derived from patients suffering from β thalassemia with Cas9 mRNA and BCL11A enhancer-2, 3, 4, 5, and 6 (i.e., enhancer-2, 3, 4, 5, and 6 in Fig. 23) sgRNAs by electroporation, then performing erythroid differentiation and detecting the cells after 12 days. Panel A is a photograph of the erythrocytes after differentiation; Panel B shows the detecting results of the expression of two membrane proteins (human CD71 and human 235a), indicating the efficiency of erythroid differentiation; wherein Mock represents cells not being genetically modified, and Enhancer-2, 3, 4, 5, and 6 represent genetically modified cells.

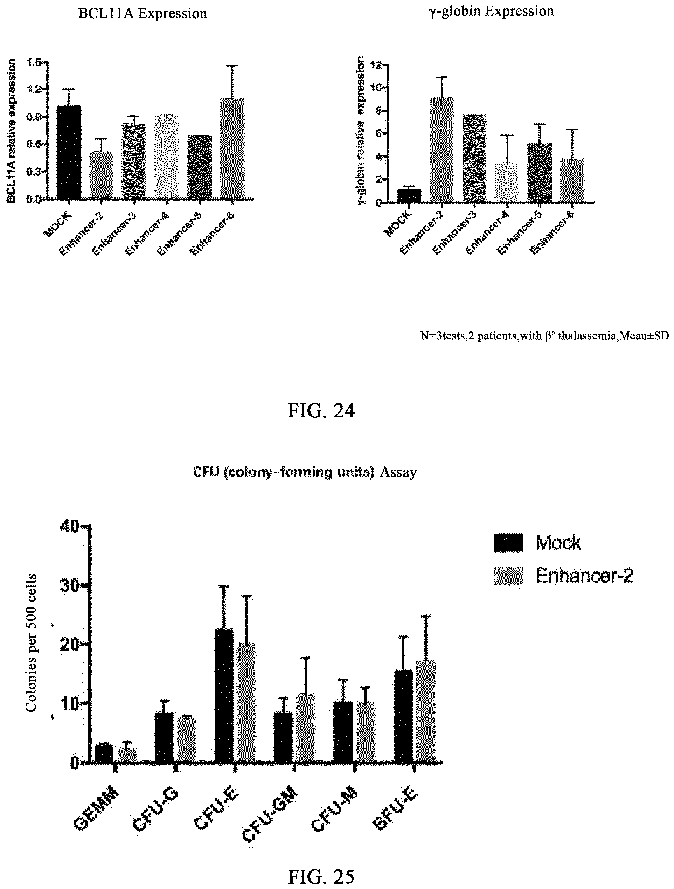

**FIG. 24****:** Graphs showing the expression of genes, which are obtained by transfecting the CD34-positive hematopoietic stem cells isolated from peripheral blood of patients suffering from β thalassemia with Cas9 mRNA and BCL11A enhancer-2, 3, 4, 5, and 6 sgRNA (i.e., enhancer-2, 3, 4, 5, and 6 in Fig. 23) by electroporation, 12 days after erythroid differentiation, detecting gene expression of BCL11A and γ-globin gene; wherein Mock represents cells not being genetically edited, and Enhancer-2, 3, 4, 5, and 6 represent genetically modified cells.
**FIG. 25****:** A graph showing the number of colonies for different blood systems, which is obtained by transfecting CD34-positive hematopoietic stem cells derived from patients suffering from β thalassemia with Cas9 mRNA and BCL11A enhancer-2 sgRNA by electroporation, performing in vitro colony-forming units assay (CFU assay) 2 days later, and then counting the number of colonies for different blood systems 14 days later; wherein BFU-E, CFU-M, CFU-GM, CFU-E, CFU-G, CFU-MM represent the cell colonies of different lineages such as the erythroid, myeloid, and lymphoid lineage, etc. in blood system.

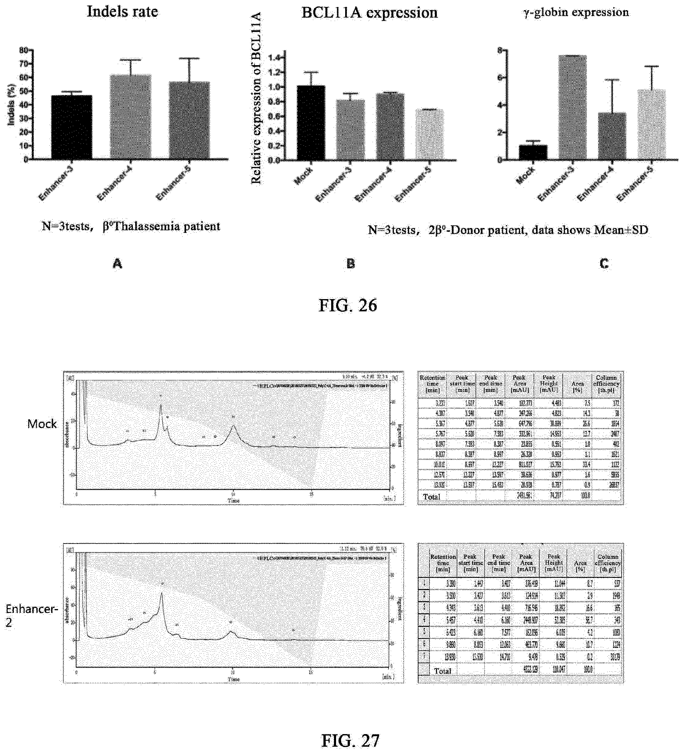

**FIG. 26****:** Graphs showing the Indels efficiency and expression of genes, which are obtained by transfecting CD34-positive hematopoietic stem cells derived from patients suffering from β thalassemia with Cas9 mRNA and BCL11A enhancer-3, -4, or -5 sgRNA by electroporation, evaluating gene editing efficiency, the gene expression of BCL11A and γ-globin gene. A) 4 days after electroporation, performing statistical analysis of the Indels efficiency produced by different sgRNAs by TIDE software; B) performing erythroid differentiation after transfecting the hematopoietic stem cells by electroporation, and detecting the gene expression of BCL11A 12 days later; C) performing erythroid differentiation after transfecting the hematopoietic stem cells by electroporation, and detecting gene expression of γ-globin gene 12days later; wherein Mock represents cells not being genetically edited, and Enhancer-3, -4, and -5 represent genetically modified cells transfected with the Enhancer-3, -4, -5 sgRNA respectively.
**FIG. 27****:** Graphs showing the results of chromatography about cells not being genetically modified (Mock) and genetically modified cells (edited with enhancer-2 sgRNA), and the expression levels of HbF and HbA.

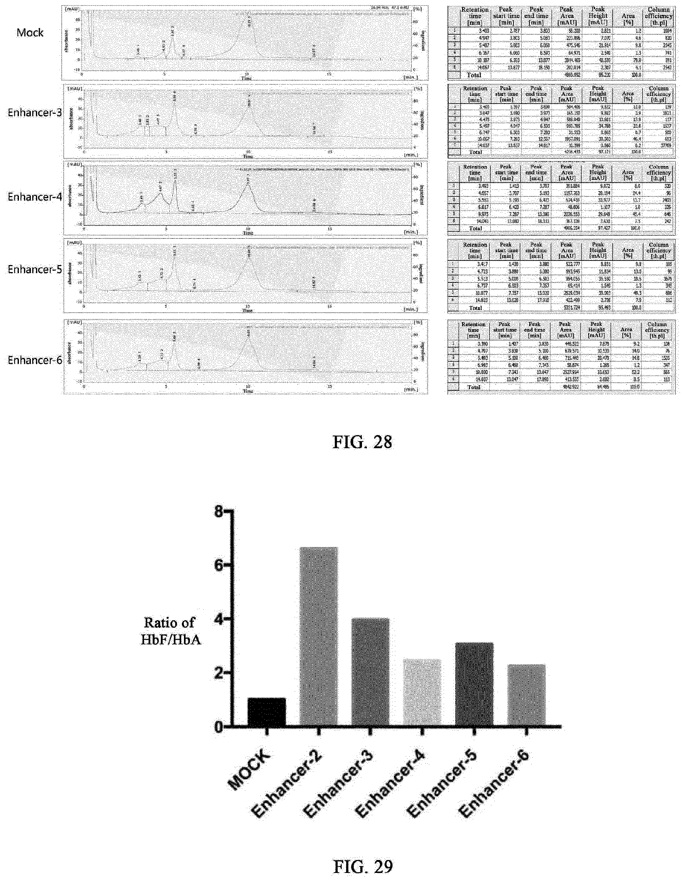

**FIG. 28****:** Graphs showing the results of chromatography about cells not being genetically edited (Mock) and genetically modified cells (edited with enhancer-3, -4, -5, and -6 sgRNA), and the expression levels of HbF and HbA.
**FIG. 29****:** A graph showing the ratios of the expression level of HbF to that of HbA calculated on the basis of the results of chromatography in Figs. 27-28.

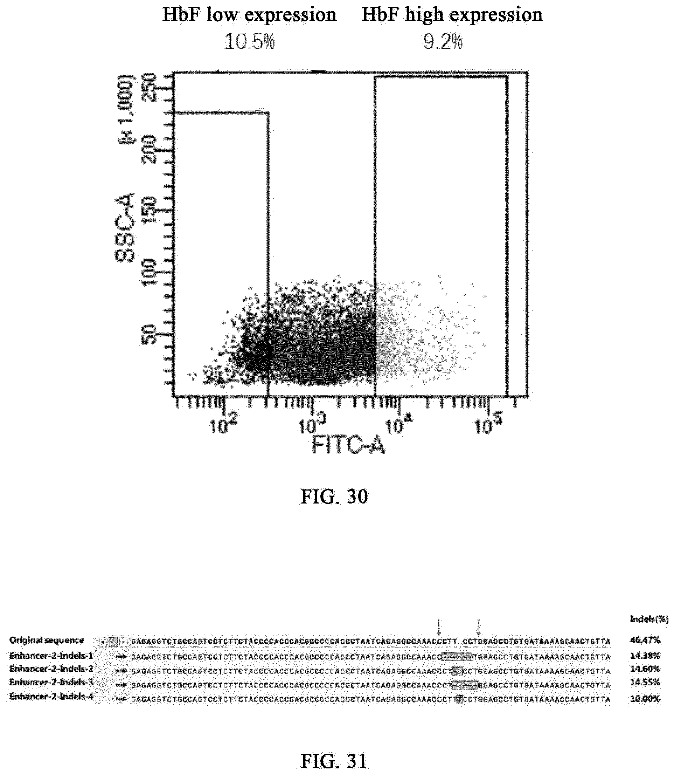

**FIG. 30****:** A graph showing the ratios of cells with high expression of HbF (about 10% ratio) and cells with low expression of HbF (about 10% ratio) after HbF staining in Example 9.
**FIG. 31****:** A graph showing the results of next-generation sequencing analysis about the extracted genomes of cells with high expression of HbF (about 10% ratio) and cells with low expression of HbF (about 10% ratio) after HbF staining in Example 9.

### DETAILED DESCRIPTION

The present application relates to a BCL11A enhancer locus for CD34-positive hematopoietic stem cells, in particular disrupting a region of the BCL11A gene from 0-15 nucleotides upstream of the CTTCCT sequence to 0-15 nucleotides downstream of the CTTCCT sequence, e.g., the 6-bp base sequence of "CTTCCT" (chr2:60495251-60495256) may eliminate the inhibition of fetal hemoglobin synthesis by BCL11A gene, thereby increasing the expression of fetal hemoglobin.

In one aspect, the present invention provides an in vitro method for increasing the expression of fetal hemoglobin (HbF) in human hematopoietic stem cells, comprising: disrupting a BCL11A genomic region from 0-15 nucleotides upstream of the CTTCCT sequence to 0-15 nucleotides downstream of the CTTCCT sequence in the chromosome 2 in the hematopoietic stem cells by gene editing technology, wherein the BCL11A genomic region is located in a region from chr2:60495236 to chr2:60495271 wherein the gene editing technology is a CRISPR/Cas9 gene editing technology,comprising introducing an sgRNA comprising a sequence selected from any one of SEQ ID NOs: 3, 4, 8 and 33-63 into the hematopoietic stem cells to edit the BCL11A genomic region, andwherein the sgRNA and a Cas9-encoding mRNA are co-introduced into the hematopoietic stem cells by electroporation.

In some embodiments, wherein the BCL11A genomic region sequence is CTTCCT.

The "CTTCCT" sequence of the BCL11A genomic region mentioned in the present application refers to a sequence located in a region from positions 60495251-60495256 of human chromosome 2, wherein a chromosomal nucleotide position is consistent with the position in the GRCh38 standard human gene sequence. Those skilled in the art will appreciate that the positions of CTTCCT sequence may be different while referring to different human genomic sequences. In some embodiments, the disruption of a BCL11A genomic region from 0-15 nucleotides upstream of the CTTCCT sequence to 0-15 nucleotides downstream of the CTTCCT sequence includes introducing "insertion and/or deletion (indel)" of a nucleotide sequence into this region, for example, introducing an indel of any kind of nucleotide (e.g., selected from A, T, C, or G) and/or quantity (e.g., 1-20, 1-15, 1-10, 1-9, 1-8, 1-7, 1-6, 1-5, 1-4, 1-3, 1-2) of nucleotides. In some embodiments, the disruption comprises replacing the original nucleotide sequence with a new nucleotide sequence, for example, replacing 1, 2, 3, 4, 5 or 6 original nucleotides of the 6 contiguous nucleotides "CTTCCT" with 1, 2, 3, 4, 5 or 6 new nucleotides. In some embodiments, the disruption comprises the knock-in or knock-out of a nucleotide sequence in a BCL11A genomic region from 0-15 nucleotides upstream of the CTTCCT sequence to 0-15 nucleotides downstream of the CTTCCT sequence, e.g., the knock-in or knock-out of a nucleotide sequence in the region of the 6 contiguous nucleotides "CTTCCT". According to the results of gene analysis, the 6-bp base sequence of "CTTCCT" contains binding sites of the STAT1 and ELF1 genes.

Without being bound by any theory or hypothesis, the disruption of CTTCCT and its vicinity in the present invention, for example, the introduction of an indel into this region, may directly affect the binding of STAT1 and ELF-1 to the BCL11A enhancer, and significantly up-regulate the expression of fetal hemoglobin, as demonstrated by Enhancer 2 in an embodiment of the invention. Gene editing around the "CTTCCT" region, for example, the gene editing a region around 1-15, 1-14, 1-13, 1-12, 1-11, 1-10, 1-9, 1-8, 1-7, 1-6, 1-5, 1-4, 1-3, 1-2 nucleotides upstream and/or downstream of the "CTTCCT" region, e.g., introducing an indel into the region may also increase the expression of fetal hemoglobin to varying degrees, see for example, Figs. 10, 23-24 of the present invention.

In some embodiments, the BCL11A genomic region disrupted in the present invention is a region from 0-15 nucleotides upstream of the CTTCCT sequence to 0-15 nucleotides downstream of the CTTCCT sequence, wherein the BCL11A genomic region is located in a region from chr2:60495236 to chr2:60495271. In some embodiments, the BCL11A genomic region disrupted in the present invention is a region from 0-14 nucleotides upstream of the CTTCCT sequence to 0-14 nucleotides downstream of the CTTCCT sequence, wherein the BCL11A genomic region is located in a region from chr2:60495237 to chr2:60495270. In some embodiments, the BCL11A genomic region disrupted in the present invention is a region from 0-13 nucleotides upstream of the CTTCCT sequence to 0-13 nucleotides downstream of the CTTCCT sequence, wherein the BCL11A genomic region is located in a region from chr2:60495238 to chr2:60495269. In some embodiments, the BCL11A genomic region disrupted in the present invention is a region from 0-12 nucleotides upstream of the CTTCCT sequence to 0-12 nucleotides downstream of the CTTCCT sequence, wherein the BCL11A genomic region is located in a region from chr2:60495239 to chr2:60495268. In some embodiments, the BCL11A genomic region disrupted in the present invention is a region from 0-11 nucleotides upstream of the CTTCCT sequence to 0-11 nucleotides downstream of the CTTCCT sequence, wherein the BCL11A genomic region is located in a region from chr2:60495240 to chr2:60495267. In some embodiments, the BCL11A genomic region disrupted in the present invention is a region from 0-10 nucleotides upstream of the CTTCCT sequence to 0-10 nucleotides downstream of the CTTCCT sequence, wherein the BCL11A genomic region is located in a region from chr2:60495241 to chr2:60495266. In some embodiments, the BCL11A genomic region disrupted in the present invention is a region from 0-9 nucleotides upstream of the CTTCCT sequence to 0-9 nucleotides downstream of the CTTCCT sequence, wherein the BCL11A genomic region is located in a region from chr2:60495242 to chr2:60495265. In some embodiments, the BCL11A genomic region disrupted in the present invention is a region from 0-8 nucleotides upstream of the CTTCCT sequence to 0-8 nucleotides downstream of the CTTCCT sequence, wherein the BCL11A genomic region is located in a region from chr2:60495243 to chr2:60495264. In some embodiments, the BCL11A genomic region disrupted in the present invention is a region from 0-7 nucleotides upstream of the CTTCCT sequence to 0-7 nucleotides downstream of the CTTCCT sequence, wherein the BCL11A genomic region is located in a region from chr2:60495244 to chr2:60495263. In some embodiments, the BCL11A genomic region disrupted in the present invention is a region from 0-6 nucleotides upstream of the CTTCCT sequence to 0-6 nucleotides downstream of the CTTCCT sequence, wherein the BCL11A genomic region is located in a region from chr2:60495245 to chr2:60495262. In some embodiments, the BCL11A genomic region disrupted in the present invention is a region from 0-5 nucleotides upstream of the CTTCCT sequence to 0-5 nucleotides downstream of the CTTCCT sequence, wherein the BCL11A genomic region is located in a region from chr2:60495246 to chr2:60495261. In some embodiments, the BCL11A genomic region disrupted in the present invention is a region from 0-4 nucleotides upstream of the CTTCCT sequence to 0-4 nucleotides downstream of the CTTCCT sequence, wherein the BCL11A genomic region is located in a region from chr2:60495247 to chr2:60495260. In some embodiments, the BCL11A genomic region disrupted in the present invention is a region from 0-3 nucleotides upstream of the CTTCCT sequence to 0-3 nucleotides downstream of the CTTCCT sequence, wherein the BCL11A genomic region is located in a region from chr2:60495248 to chr2:60495259. In some embodiments, the BCL11A genomic region disrupted in the present invention is a region from 0-2 nucleotides upstream of the CTTCCT sequence to 0-2 nucleotides downstream of the CTTCCT sequence, wherein the BCL11A genomic region is located in a region from chr2:60495249 to chr2:60495258. In some embodiments, the BCL11A genomic region disrupted in the present invention is a region from 0-1 nucleotide upstream of the CTTCCT sequence to 0-1 nucleotide downstream of the CTTCCT sequence, wherein the BCL11A genomic region is located in a region from chr2:60495250 to chr2:60495257. In some embodiments, the BCL11A genomic region sequence disrupted in the present invention is CTTCCT, wherein the BCL11A genomic region is located in a region from chr2:60495251 to chr2:60495256.

In some embodiments, the utmost upstream of the BCL11A genomic region is located at the 15th, 14th, 13th, 12th, 11th, 10th, 9th, 8th, 7th, 6th, 5th, 4th, 3rd, 2nd, or 1st nucleotide upstream of the CTTCCT sequence, or the 1st, 2nd, or 3rd nucleotide at the 5' end of the CTTCCT sequence. In some embodiments, the utmost downstream of the BCL11A genomic region is located at the 15th, 14th, 13th, 12th, 11th, 10th, 9th, 8th, 7th, 6th, 5th, 4th, 3rd, 2nd, or 1st nucleotide downstream of the CTTCCT sequence, or the 1st, 2nd, or 3rd nucleotide at the 3' end of the CTTCCT sequence. In some embodiments, the utmost upstream of the BCL11A genomic region is located at chr2:60495253, chr2:60495252, chr2:60495251, chr2:60495250, chr2:60495249, chr2:60495248, chr2:60495247, chr2:60495246, chr2:60495245, chr2:60495244, chr2:60495243, chr2:60495242, chr2:60495241, chr2:60495240, chr2:60495239, chr2:60495238, chr2:60495237, or chr2:60495236. In some embodiments, the utmost downstream of the BCL11A genomic region is located at chr2:60495254, chr2:60495255, chr2:60495256, chr2:60495257, chr2:60495258, chr2:60495259, chr2:60495260, chr2:60495261, chr2:60495262, chr2:60495263, chr2:60495264, chr2:60495265, chr2:60495266, chr2:60495267, chr2:60495268, chr2:60495269, chr2:60495270, or chr2:60495271. The utmost upstream and utmost downstream of the BCL11A genomic region may be a combination of any one of the above utmost upstream and utmost downstream positions, and the BCL11A genomic region is not a GATAA site.

In some embodiments, the BCL11A genomic region is selected from the sequences with 1-36 (e.g., about 1-4, 1-6, 1-8, 1-10, 1-15, 1-20, 1-25, or 1-30) contiguous nucleotides of SEQ ID NO: 31 or SEQ ID NO: 32 as shown below, and the BCL11A genomic region is not a GATAA site:
SEQ ID NO: 31 (a nucleotide sequence from chr2:60495236 to chr2:60495271) atcagaggccaaacccttcctggagcctgtgataaa
SEQ ID NO: 32 (a complementary sequence of the nucleotide sequence from chr2:60495236 to chr2:60495271)
   tttatcacaggctccaggaagggtttggcctctgat

Accordingly, in one aspect, the present invention relates to an in vitro method for increasing the expression of fetal hemoglobin (HbF) in human hematopoietic stem cells, comprising: disrupting a BCL11A genomic region of 6 consecutive bases "CTTCCT" (chr2:60495251- 60495256) in the chromosome 2 in the hematopoietic stem cells by gene editing technology wherein the gene editing technology is a CRISPR/Cas9 gene editing technology,comprising introducing an sgRNA comprising a sequence selected from any one of SEQ ID NOs: 3, 4, 8 and 33-63 into the hematopoietic stem cells to edit the BCL11A genomic region, andwherein the sgRNA and a Cas9-encoding mRNA are co-introduced into the hematopoietic stem cells by electroporation.

The invention also relates to an in vitro method for increasing the expression of fetal hemoglobin (HbF) in human hematopoietic stem cells, comprising: disrupting a region around 1-15, 1-14, 1-13, 1-12, 1-11, 1-10, 1-9, 1-8, 1-7, 1-6, 1-5, 1-4, 1-3, 1-2 nucleotides upstream and/or downstream of the 6 consecutive bases CTTCCT region in the chromosome 2 in the hematopoietic stem cells by gene editing technology wherein the gene editing technology is a CRISPR/Cas9 gene editing technology,comprising introducing an sgRNA comprising a sequence selected from any one of SEQ ID NOs: 3, 4, 8 and 33-63 into the hematopoietic stem cells to edit the BCL11A genomic region, andwherein the sgRNA and a Cas9-encoding mRNA are co-introduced into the hematopoietic stem cells by electroporation.

As disclosed herein, the method comprises introducing an sgRNA targeting the BCL11A genomic region from 0-15 nucleotides upstream of the CTTCCT sequence to 0-15 nucleotides downstream of the CTTCCT sequence (e.g., an sgRNA comprising CTTCC or a sequence complementary to CTTCC) into the hematopoietic stem cells to achieve editing of the BCL11A genome, wherein the BCL11A genomic region is located in a region from chr2:60495236 to chr2:60495271. In the invention, the method comprises introducing an sgRNA comprising a sequence selected from any one of SEQ ID NOs: 3, 4, 8 and 33-63 into the hematopoietic stem cells to achieve editing of the BCL11A genome.

Also disclosed is an sgRNA (such as a chemically modified sgRNA) required for gene editing of the hematopoietic stem cells, comprising the sgRNA targeting the BCL11A genomic region from 0-15 nucleotides upstream from the CTTCCT sequence to 0-15 nucleotides downstream of the CTTCCT sequence, wherein the BCL11A genomic region is located in a region from chr2:60495236 to chr2:60495271, for example, comprising CTTCC or a nucleotide sequence complementary to CTTCC, and further, comprising a nucleotide sequence selected from any one of SEQ ID NOs: 3, 4, 8 and 33-63.

In some embodiments the method is used *in vitro* for gene editing of the hematopoietic stem cells of patients with β thalassemia and sickle cell anemia.

In some embodiments, the disrupted BCL11A genomic region in the hematopoietic stem cells, mature erythrocytes, or precursor cells of the mature erythrocytes comprises any one of the sequences listed in Fig. 31, such as Enhancer-2-Indels-1, 2, 3, or 4.

One object of the present invention is to efficiently modify the gene of CD34⁺ hematopoietic stem cells by CRISPR/Cas gene editing technology. For example, CD34-positive hematopoietic stem cells may be obtained from umbilical cord blood or bone marrow, then gene editing is performed by introducing Cas9 and a given sgRNA into the hematopoietic stem cells under optimized electroporation conditions, and the genetically edited hematopoietic stem cells are transplanted into experimental animals for the evaluation of the ability of the hematopoietic stem cells to reconstitute the hematopoietic system. The sgRNA may be designed for targeting BCL11A enhancers, such as targeting BCL11A (+58) locus, by a variety of design software, such as the "CRISPR RGEN TOOLS" software. In some instances, the designed sgRNA is chemically modified, such as a 2'-O-methyl modification and an internucleotide 3'-thio modification at the three bases of its 5'-end and 3'-end. High efficient sgRNA may be obtained by testing the combinations of Cas9 mRNA and different sgRNAs. In some embodiments, the hematopoietic stem cells are obtained by clinical grade magnetic column sorting, and the Cas9 protein encoding mRNA and the chemically modified sgRNA for gene editing are obtained by in vitro transcription assays. The genetically modified hematopoietic stem cells are differentiated into erythroid lineage, and the erythropoiesis rate is detected. For example, the genetically modified hematopoietic stem cells may be transplanted into NPG mice irradiated by irradiator for the evaluate of the ability to reconstitute rebuilding the hematopoietic system; alternatively, the CD34-positive hematopoietic stem cells of patients may be separated for the evaluation of gene editing efficiency, the ability of erythroid differentiation, the expression of γ-globin and fetal hemoglobin.

Firstly, the present invention provides an in vitro method for improving the expression of fetal hemoglobin (HbF), comprising: performing gene editing of an enhancer locus of BCL11A in the hematopoietic stem cells by a CRISPR/Cas9 gene editing technology, comprising introducing an sgRNA comprising a sequence selected from any one of SEQ ID NOs: 3, 4, 8 and 33-63 into the hematopoietic stem cells to edit the BCL11A genomic region, andwherein the sgRNA and a Cas9-encoding mRNA are co-introduced into the hematopoietic stem cells by electroporation. The editing process designs an sgRNA targeting the enhancer locus sequence of BCL11A in hematopoietic stem cells. The enhancer loci of BCL11A are named as loci +62, +58, and +55, according to their distances (numbers of kilobase) from the transcription start site. It is reported that the erythroid enhancer of BCL11A gene negatively regulates the expression of fetal hemoglobin (HbF), wherein the region containing the positions with a distance of 55 kb (kb represents 1000 bases), 58 kb, and 62 kb from the transcription start site is a key regulatory region. Although researchers have studied the loci +55, +58, and +62, the length of gene sequences before and after the above three loci are all above 1000 bp, with a total of about 6000 bp, and thus it is unknown to those skilled in the art which specific region may be edited to achieve the desired editing effect, even for locus +58, the efficiency of gene editing varies greatly (see: Bauer, DE et al. An erythroid enhancer of BCL11A subject to genetic variation decided fetal hemoglobin level. Science. 342, 253-257 (2013), and Canver MC, et al., BCL11A enhancer dissection by Cas9-mediated in situ saturating mutagenesis. Nature. 2015, Nov. 12; 527 (7577):192-7). Therefore, the first problem for a person skilled in the art is to find a specific area which is critical to the efficiency of gene editing.

With intensive research the inventors of the present application find that the efficiency of gene editing is greatly affected by a 150-bp base sequence at locus +58 (located in a region from positions 60495197 to 60495346 on human chromosome 2, abbreviated herein as chr2:60495197-60495346, the sequence is, for example, as shown in SEQ ID NO: 2), wherein the most critical region is the 6-bp base sequence region of "CTTCCT". Through the disruption of this region, the expression of fetal hemoglobin may be increased significantly and efficiently. The disruption of the BCL11A genomic region from 0-15 nucleotides upstream of the CTTCCT sequence to 0-15 nucleotides downstream of the CTTCCT sequence, such as around a region upstream and/or downstream of the CTTCCT sequence 1-15, 1-14, 1-13 1-12, 1-1-1, 1-10, 1-9, 1-8, 1-7, 1-6, 1-5, 1-4, 1-3, 1-2 nucleotides, can also increase the expression of fetal hemoglobin to varying degrees.

All the sgRNAs provided may be used to efficiently realize gene editing of the hematopoietic stem cells derived from a normal donor and a patient suffering from anemia, wherein the candidate sgRNAs with relatively higher efficiency may significantly increase the expression of fetal hemoglobin. It was reported in literatures that, through designing sgRNAs libraries for loci +55, +58, and +62, and screening with cell models by using CRISPR/Cas9, a sequence targeting "GATAA" at locus +58 is find to be a key regulatory sequence, i.e., an sgRNA comprising the "GATAA" sequence is most effective in increasing fetal hemoglobin. However, it is worth noting that the region around +58k anchored by the sgRNA being discovered herein is different from the critical base site of "GATAA" disclosed in the prior art.

The inventors of the present invention found that the sequence "CTTCCT" in the BCL11A genomic region (the position pointed by the arrows in Fig. 31) is a key target sequence. If this sequence is disrupted in the gene-edited cells, the expression of fetal hemoglobin may be significantly up-regulated. According to the results of the genetic analysis, the 6-bp base sequence of "CTTCCT" contains the binding sites of the STAT1 and ELF1 genes. According to the literature, wherein the binding site of STAT1 is the motif of "TTCCnGGA" (n stands for any base) (Raja, et al. Nucleic Acids Research. 2008; George, et al., Journal of Biological Chemistry. 2001; Christoph, et al., Plos one. 2010), and the binding site of ELF-1 is the motif of "TTCC" (Steven, et al., Scientific Reports, 2015; Yuang-Taung Juang, et al., The Journal of Immunology, 2007). The gene editing of introducing the sgRNA significantly increases the expression of fetal hemoglobin, and is sufficient for use in clinical treatment.

Those skilled in the art can understand that after acquiring a high-efficient gene editing region, a person skilled in the art can use any gene editing method, such as zinc finger nuclease-based gene editing technology, TALEN gene editing technology, and CRISPR/Cas (such as CRISPR/Cas9) gene editing technology, and other gene editing methods found in the future, to perform gene editing of the acquired high-efficiency gene editing region, optimize the gene editing conditions, and achieve efficient gene editing. Thus, the disclosure encompasses any technical solutions for performing gene editing with a BCL11A gene targeting sequence selected from SEQ ID NOs: 3-25 and 33-63 and identified herein through any available gene editing method. The disclosure relates to a technical solution for achieving efficient gene editing by the CRISPR/Cas9 gene editing technology.

As used herein, "CRISPR/Cas" is a gene editing technology including, but not limited to, a variety of naturally occurring or artificially designed CRISPR/Cas systems, such as the CRISPR/Cas9 system. The naturally occurring CRISPR/Cas system is an adaptive immune defense formed in bacteria and archaea during long-term evolution, and is used to fight against the invading viruses and foreign DNAs. For example, the mechanism of CRISPR/Cas9 is the fact that crRNA (CRISPR-derived RNA) binds to tracrRNA (trans-activating RNA) through base pairing to form a tracrRNA/crRNA complex, which directs the nuclease Cas9 protein to cleave the double-stranded DNA at a target site in a sequence matching with the crRNA. However, a single guide RNA (sgRNA) with the guiding ability may be formed by transformation with artificially designed tracrRNA and the crRNA, the sgRNA is able to guide Cas9 to perform site-directed cleavage of DNA. As an RNA-guided dsDNA-binding protein, the Cas9 effector nuclease is capable of co-localizing RNA, DNA and proteins, thereby providing tremendous potential to be engineered. The CRISPR/Cas system may use Cas proteins of type I, II, or III. In the invention, the method uses Cas9. Other suitable CRISPR/Cas systems include, but are not limited to, the systems and methods described in WO2013176772, WO2014065596, WO2014018423, and US8,697,359.

In the present invention, an "sgRNA" and a "gRNA" may be used interchangeably as a "single guide RNA", "synthetic guide RNA" or a "guide RNA". The sgRNA comprises a guide sequence targeting a sequence of interest. In a preferred embodiment, the sgRNA further comprises a tracr sequence and a tracr chaperone sequence.

A "guide sequence" in the present invention may be a sequence of about 17-20 bp with specified targeting site, and may be used interchangeably with a "leader sequence" or a "spacer". In the context of the formation of a CRISPR complex, a "target sequence" is, for example, a sequence that a guide sequence is designed to be complementary thereto, wherein hybridization between a target sequence and the guide sequence promotes the formation of a CRISPR complex, and the hybridization requires " that the "target sequence" is sufficiently complementary to the "guide sequence" or "leader sequence", as long as it can cause hybridization and promote the formation of the CRISPR complex, while it is not necessary to be completely complementary.

"Complementary" means a "guide sequence" or "leader sequence" may hybridize to a target nucleotide sequence (herein the present invention, a target nucleotide sequence of BCL11A genome in the hematopoietic stem cells) according to the nucleotide pairing principle found by Watson and Crick. Those skilled in the art will appreciate that a "guide sequence" may hybridize to a target nucleotide sequence as long as it is sufficiently complementary to the target nucleotide sequence, and complete (100% of) complementation between them is not necessary. In some embodiments, the degree of complementarity between the guide sequence and its corresponding target sequence may be about or greater than about 75%, 80%, 85%, 90%, 91%, 92%, 93%, 94%, 95%, 96%, 97%, 98%, 99% or more, when optimal alignment is performed by using an appropriate alignment algorithm. The optimal alignment may be determined by using any suitable algorithm for aligning sequences, including the Smith-Waterman algorithm, the Needleman-Wimsch algorithm, the Burrows-Wheeler Transform based algorithm, and the like.

Typically, in the context of an endogenous CRISPR system, the formation of a CRISPR complex (including the hybridization of a guide sequence to a target sequence, and then complexing with one or more Cas proteins) results in the cleavage of one or two strands in or near the target sequence (e.g., within the range of 1, 2, 3, 4, 5, 6, 7, 8, 9, 10, 20, 50 or more base pairs from the target sequence). Without wishing to be bound by theory, the tracr sequence may comprise all or a portion of a wild-type tracr sequence (for example, about or greater than about 20, 23, 26, 29, 32, 35, 38, 41, 44, 47, 50, 53, 56, 59, 62, 65, 70, 75, 80, 85 or more nucleotides of the wild-type tracr sequence), or a tracr sequence consisting of the above may also form part of a CRISPR complex, for example, by hybridizing along at least a portion of a tracr sequence to all or a portion of the tracr chaperone sequence which is operably linked to the guide sequence.

In some embodiments, the tracr sequence is sufficiently complementary to the tracr chaperone sequence to hybridize and participate in the formation of a CRISPR complex. Similar to the case of hybridizing a "target sequence" to a "guide sequence" or a "leader sequence", complete complementation is not necessary as long as it is sufficient to perform its function. In some embodiments, in the case of optimal alignment, the tracr sequence has at least 50%, 60%, 70%, 80%, 90%, 95% or 99% complementarity along the length of the tracr chaperone sequence.

In the present application, the sgRNA sequence targeting the BCL11A genomic region comprises an sgRNA complementary to at least 17, preferably 18, preferably 19, or preferably 20 contiguous nucleotides of the BCL11A genomic region. In some embodiments, a sequence targeting the BCL11A locus of the hematopoietic stem cells is represented by SEQ ID NO: 31 or 32, and an sgRNA is designed for the base region represented by SEQ ID NO: 31 or 32, the sgRNA sequence is required to be complementary to a sequence of at least 17, preferably 18, preferably 19, or preferably 20 contiguous nucleotides of SEQ ID NO: 31 or 32.

Specifically, the following table lists the sgRNA sequences targeting the BCL11A genomic region from 0-15 nucleotides upstream of the CTTCCT sequence to 0-15 nucleotides downstream of the CTTCCT sequence.

| **SEQ ID NO:** | **sgRNA sequences** |
|---|---|
| 3 | cacaggctccaggaagggtt |
| 4 | atcagaggccaaacccttcc |
| 8 | tttatcacaggctccaggaa |
| 33 | tcagaggccaaacccttcct |
| 34 | cagaggccaaacccttcctg |
| 35 | agaggccaaacccttcctgg |
| 36 | gaggccaaacccttcctgga |
| 37 | aggccaaacccttcctggag |
| 38 | ggccaaacccttcctggagc |
| 39 | gccaaacccttcctggagcc |
| 40 | ccaaacccttcctggagcct |
| 41 | caaacccttcctggagcctg |
| 42 | aaacccttcctggagcctgt |
| 43 | aacccttcctggagcctgtg |
| 44 | acccttcctggagcctgtga |
| 45 | cccttcctggagcctgtgat |
| 46 | ccttcctggagcctgtgata |
| 47 | cttcctggagcctgtgataa |
| 48 | ttcctggagcctgtgataaa |
| 49 | ttatcacaggctccaggaag |
| 50 | tatcacaggctccaggaagg |
| 51 | atcacaggctccaggaaggg |
| 52 | tcacaggctccaggaagggt |
| 53 | acaggctccaggaagggttt |
| 54 | caggctccaggaagggtttg |
| 55 | aggctccaggaagggtttgg |
| 56 | ggctccaggaagggtttggc |
| 57 | gctccaggaagggtttggcc |
| 58 | ctccaggaagggtttggcct |
| 59 | tccaggaagggtttggcctc |
| 60 | ccaggaagggtttggcctct |
| 61 | caggaagggtttggcctctg |
| 62 | aggaagggtttggcctctga |
| 63 | ggaagggtttggcctctgat |

The present disclosure provides an in vitro method for increasing the expression of fetal hemoglobin (HbF) in human hematopoietic stem cells, comprising: disrupting a BCL11A genomic region from chr2:60495236 to chr2:60495255, particularly position 60495238 in the chromosome 2 in the hematopoietic stem cells by gene editing technology. The gene editing technology is a zinc finger nuclease-based gene editing technology, a TALEN gene editing technology, or a CRISPR/Cas gene editing technology, preferably a CRISPR/Cas9 gene editing technology, preferably the target nucleotide sequence in the BCL11A genome is complementary to a leader sequence of the sgRNA comprising SEQ ID NO: 4. The method of the present invention involves introducing an sgRNA comprising the sequence of SEQ ID NO: 4 into the hematopoietic stem cells to achieve editing of the BCL11A genome, co-introducing the sgRNA and Cas9-encoding mRNA into the hematopoietic stem cells by electroporation, preferably under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two, and/or three bases at the 5' end and/or the last base at the 3' end of the sgRNA. The present disclosure provides an in vitro method for increasing the expression of fetal hemoglobin (HbF) in human hematopoietic stem cells, comprising: disrupting a BCL11A genomic region from chr2:60495247 to chr2:60495266, particularly position 60495263 in the chromosome 2 in the hematopoietic stem cells by gene editing technology. The gene editing technology is a zinc finger nuclease-based gene editing technology, a TALEN gene editing technology, or a CRISPR/Cas gene editing technology, preferably a CRISPR/Cas9 gene editing technology, preferably the target nucleotide sequence in the BCL11A genome is complementary to a leader sequence of the sgRNA comprising SEQ ID NO: 3. The method of the present invention involves introducing an sgRNA comprising the sequence of SEQ ID NO: 3 into the hematopoietic stem cells to achieve editing of the BCL11A genome, co-introducing the sgRNA and Cas9-encoding mRNA into the hematopoietic stem cells by electroporation, preferably under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two, and/or three bases at the 5' end and/or the last base at the 3' end of the sgRNA.

The present disclosure provides an in vitro method for increasing the expression of fetal hemoglobin (HbF) in human hematopoietic stem cells, comprising: disrupting a BCL11A genomic region from chr2:60495252 to chr2:60495271, particularly position 60495268 in the chromosome 2 in the hematopoietic stem cells by gene editing technology. The gene editing technology is a zinc finger nuclease-based gene editing technology, a TALEN gene editing technology, or a CRISPR/Cas gene editing technology, preferably a CRISPR/Cas9 gene editing technology, preferably the target nucleotide sequence in the BCL11A genome is complementary to a leader sequence of the sgRNA comprising SEQ ID NO:8. The method of the present invention involves introducing an sgRNA comprising the sequence of SEQ ID NO: 8 into the hematopoietic stem cells to achieve editing of the BCL11A genome, co-introducing the sgRNA and Cas9-encoding mRNA into the hematopoietic stem cells by electroporation, preferably under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two, and/or three bases at the 5' end and/or the last base at the 3' end of the sgRNA.

In here, the inventors designed 23 sgRNAs targeting the BCL11A enhancer. The following table lists the positions of the genomic sequences on human chromosome 2 which are targeted by all the 23 sgRNAs, as well as the Cas9 cleavage sites triggered by each sgRNA.

| **Names** | **Positions on the human genomic sequence** | **Cleavage sites** |
|---|---|---|
| BCL11A enhancer-7 | chr2:60495203-60495222 | chr2:60495219 |
| BCL11A enhancer-8 | chr2:60495208-60495227 | chr2:60495224 |
| BCL11A enhancer-9 | chr2:60495217-60495236 | chr2:60495233 |
| BCL11A enhancer-10 | chr2:60495218-60495237 | chr2:60495234 |
| BCL11A enhancer-11 | chr2:60495219-60495238 | chr2:60495235 |
| BCL11A enhancer-14 | chr2:60495221-60495240 | chr2:60495223 |
| BCL11A enhancer-12 | chr2:60495222-60495241 | chr2:60495238 |
| BCL11A enhancer-13 | chr2:60495223-60495242 | chr2:60495239 |
| BCL11A enhancer-15 | chr2:60495228-60495247 | chr2:60495244 |
| BCL11A enhancer-16 | chr2:60495229-60495248 | chr2:60495245 |
| BCL11A enhancer-17 | chr2:60495230-60495249 | chr2:60495246 |
| BCL11A enhancer-18 | chr2:60495231-60495250 | chr2:60495247 |
| BCL11A enhancer-19 | chr2:60495234-60495253 | chr2:60495250 |
| BCL11A enhancer-20 | chr2:60495235-60495254 | chr2:60495251 |
| BCL11A enhancer-2 | chr2:60495236-60495255 | chr2:60495238 |
| BCL11A enhancer-1 | chr2:60495247-60495266 | chr2:60495263 |
| BCL11A enhancer-6 | chr2:60495252-60495271 | chr2:60495268 |
| BCL11A enhancer-5 | chr2:60495253-60495272 | chr2:60495269 |
| BCL11A enhancer-4 | chr2:60495257-60495276 | chr2:60495273 |
| BCL11A enhancer-3 | chr2:60495264-60495283 | chr2:60495280 |
| BCL11A enhancer-22 | chr2:60495299-60495318 | chr2:60495301 |
| BCL11A enhancer-23 | chr2:60495319-60495338 | chr2:60495335 |
| BCL11A enhancer-21 | chr2:60495320-60495339 | chr2:60495336 |

Analysis of the cleavage sites of the above 23 sgRNAs reveals that, the Cas9 cleavage sites triggered by these sgRNAs are concentrated in the genomic region from chr2:60495219 to chr2:60495336 in the BCL11A gene. And the efficiency produced by the sgRNA targeting a region from 0-15 nucleotides upstream of the CTTCCT sequence to 0-15 nucleotide regions downstream of the CTTCCT sequence (e.g., the CTTCCT) is the best, and the off-target effect of said sgRNA is relatively smaller, i.e., the sgRNA may be used for efficient gene editing.

Further, in the present disclosure, the sgRNAs designed by the inventors can efficiently produce Indels, and a preferred sgRNA is selected from any one of SEQ ID NOs: 3, 4, 8, and 33-63.

A particular instance further comprises a composition containing said sgRNA, comprising any one of the above mentioned sgRNAs or the vector thereof.

In general, the guide sequence in an sgRNA is any polynucleotide sequence sufficiently complementary to a target polynucleotide sequence and capable of hybridizing to the target sequence, thereby guiding the sequence-specific binding of the CRISPR complex to the target sequence. In some instances, when optimal alignment is performed by using an appropriate alignment algorithm, the degree of complementarity between the guide sequence and its corresponding target sequence is about or greater than about 80%, 85%, 90%, 95%, 97.5%, 99% or more. Optimal alignment may be determined by using any suitable algorithm for aligning sequences, non-limiting examples of the algorithms include: the Smith-Waterman algorithm, the Needleman-Wimsch algorithm, the Burrows-Wheeler Transform based algorithm (e.g., Burrows Wheeler Aligner), ClustalW, Clustai X, BLAT, Novoalign (Novocraft Technologies, ELAND (Illumina, San Diego, CA), SOAP (available at soap.genomics.org.cn), and Maq (available at maq.sourceforge.net). In some instances, the length of the guide sequence may be about or greater than about 10, 11, 12, 13, 14, 15, 16, 17, 18, 19, 20, 21, 22, 23, 24, 25, 26, 27, 28, 29, 30, 35, 40, 45, 50, 55, 60, 65, 70, 75 or more nucleotides. In some embodiments, the length of the guide sequence is less than about 75, 70, 65, 60, 55, 50, 45, 40, 35, 30, 25, 20, 15, 12 or fewer nucleotides. The ability of the guide sequence to direct sequence-specific binding of the CRISPR complex to the target sequence may be assessed by any suitable assay. For example, a host cell having a corresponding target sequence may be provided with the components of a CRISPR system (including the guide sequences to be tested) capable of forming a CRISPR complex, for example, it may be performed by transfection with a vector encoding the components of the CRISPR sequences, followed by evaluation of preferential cleavages within the target sequence (e.g., determined by Surveyor as described herein). Similarly, the cleavage of a target polynucleotide sequence may be assessed in a test tube by providing a target sequence and the components of a CRISPR complex (containing a guide sequence to be tested, and a control guide sequence different from the guide sequence), and then comparing the binding or cleavage rate of the tested guide sequence and the control guide sequence on the target sequence. The above assay and evaluation can also be carried out by using other detection methods known to those skilled in the art.

In the present invention, the sgRNAs used in performing gene editing are preferably chemically modified. "chemically modified sgRNA" refers to an sgRNA modified with a specific chemical group, for example, 2'-O-methyl modification and/or an internucleotide 3'-thio modification at the 5' end and 3' end three bases.

The chemically modified sgRNA adopted by the present inventors is considered to have the following two advantages. First, since sgRNA is a single-strand RNA, its half-life is very short, and it will degrade rapidly after entering the cell (not more than 12 hrs), while at least 48 hrs is needed for Cas9 protein to bind sgRNA and perform gene editing. Therefore, a chemically modified sgRNA is used for stable expression after entering the cell, and after binding to the Cas9 protein it can efficiently edit the genome to generate Indels. Secondly, an unmodified sgRNA has poor ability to penetrate cell membranes and cannot effectively enter cells or tissues to perform corresponding functions, while the ability of a chemically modified sgRNA to penetrate cell membranes is generally enhanced. Chemical modification methods commonly used in the art may be employed in the present invention, as long as the stability of the sgRNA may be improved (prolonged half-life) and the ability to enter the cell membrane may be enhanced. In addition to the specific chemical modifications used in the examples, other modification methods are also included, for example, those chemical modification methods reported in the following literatures: Deleavey G.F., Damha MJ.; Designing chemically modified oligonucleotides for targeted gene silencing. Chem Biol. 2012, Aug 24; 19(8): 937-954, and Hendel et al.; Chemically modified guide RNAs enhance CRISPR-Cas genome editing in human primary cells. Nat Biotechnol., 2015 Sep; 33(9): 985-989.

The sgRNA and Cas9-encoding mRNA are introduced into the hematopoietic stem cells by electroporation, e.g., under the electroporation conditions such as 250-360 V, 0.5-1 ms; 250-300 V, 0.5-1 ms; 250 V, 1 ms; 250 V 2ms; 300V, 0.5ms; 300V, 1ms; 360V, 0.5ms; or 360V, 1ms. The sgRNA and Cas9-encoding mRNA are co-introduced into the hematopoietic stem cells by electroporation. In some embodiments, the Cas9-encoding nucleotides is an mRNA comprising an ARCA cap. In some embodiments, the Cas9-encoding mRNA is included in a viral vector, such as a lentiviral vector. In some embodiments, the Cas9-encoding mRNA comprises the sequence represented by SEQ ID NO:26. In some embodiments, the sgRNA and the Cas9-encoding mRNA are included in the same vector.

In some aspects, the disclosure provides in vitro methods of delivering an sgRNA and/or Cas9-encoding mRNA to hematopoietic stem cells. In some instances, in this delivery the construct may be introduced into the hematopoietic stem cells or other host cells by using conventional viral based and non-viral based gene transferring methods. Non-viral delivery systems include DNA plasmids, RNA (such as the vector transcripts described herein), naked nucleic acids, and liposomes. Viral vector delivery systems include DNA and RNA viruses having a free or integrated genome for the delivery to a cell. The inventors introduce the genes encoding Cas9 and the sgRNA into the hematopoietic stem cells by electroporation to perform gene editing of a specific sequence of the BCL11A genomic region in the hematopoietic stem cells. After repeated experiments, the inventors found that the gene editing efficiency for co-introducing Cas9-coding mRNA and the sgRNA into the hematopoietic stem cells by electroporation under the conditions of 200-600 V, 0.5-2 ms is significantly higher than that under other electroporation conditions.

In some embodiments, the chemically modified sgRNA and the Cas9-encoding gene are co-introduced into the CD34+ hematopoietic stem cells by electroporation, resulting in a high gene editing efficiency (indicated as Indels %). The data in the examples shows that, if the Cas9 mRNA is co-introduced together with a chemically unmodified sgRNA by electroporation, the Indels efficiency is only 2.7%, and it is much lower than that obtained by introducing a chemically modified sgRNA by electroporation (the efficiency is at least 10% or above).

As used herein, the full name of "Indel" is an insertion/deletion, i.e., an insertion and deletion mutation.

As used herein, "hematopoietic stem cells (hematopoietic stem and progenitor cells, HSPCs)" are the most primitive hematopoietic cells for generating various blood cells. Their main characteristics are the strong proliferation potential, multi-directional differentiation ability and self-renewal ability. Therefore, they not only differentiates into and supplements various blood cells, but also maintains the characteristics and quantity of stem cells through self-renewal. Hematopoietic stem cells have different differentiation degrees and proliferation abilities, and are heterogeneous. Pluripotent hematopoietic stem cells are the most primitive, they firstly differentiate into committed pluripotent hematopoietic stem cells, such as myeloid hematopoietic stem cells capable of producing granulocytic, erythroid, mononuclear cells and megakaryocyte-platelet linenages, and lymphoid stem cells capable of producing B and T lymphocytes. These two types of stem cells maintain the basic characteristics of hematopoietic stem cells, but they are slightly differentiated, and respectively responsible for producing the "myeloid components" and the lymphocytes, so they are called as "committed pluripotent hematopoietic stem cells". They further differentiate into hematopoietic progenitor cells, which are also primitive blood cells, but have lost many of the basic features of hematopoietic stem cells, e.g., losing the ability of multi-directional differentiation, and they can only differentiate into one cell line or two closely related cell lines; they lose the ability of repeated self-renewal, and their quantity is supplemented by the proliferation and differentiation of hematopoietic stem cells; and the proliferation potential is limited and they can only divide several times. According to the number of blood cell lines that may be differentiated from the hematopoietic progenitor cells, they may be divided into unipotent hematopoietic progenitor cells (differentiating into only one blood cell line) and oligopotent hematopoietic progenitor cells (differentiating into two or three blood cell lines). The term "hematopoietic stem cells" according to the present invention encompasses pluripotent hematopoietic stem cells, committed pluripotent hematopoietic stem cells, and hematopoietic progenitor cells, and it is a generic term for all hematopoietic stem cells having different heterogeneities.

In a specific embodiment of the present invention, the hematopoietic stem cells (HSPCs) used in the present invention for gene editing may be derived from bone marrow, umbilical cord blood, or peripheral blood mononuclear cells (PBMCs).

As used herein, "CRISPR RGEN" is the name of a website developed by the research team of Korean scientist Jin-Soo Kim specifically for designing sgRNA at www.rgenome.net/about/.

As used herein, "TIDE" is the name of a tool website for analyzing Indels efficiency at tide-calculator.nki.nl.

As used herein, "CD34, CD45RA, CD3, CD4, CD8, CD33, CD19, CD56, CD71, and CD235a" are membrane protein markers of blood system cells.

As used herein, "BCL11A" is a transcription factor first discovered in mice as a binding site for retroviruses, it was named Evi9 and was later found in the human genome locating at locus 2p13 of the short arm in the chromosome 2, and it is mainly expressed in the germinal center of B lymphocytes.

As used herein, "HBB/HBG" are different subtypes of hemoglobin. Hemoglobin is a protein responsible for carrying oxygen in higher organisms. Hemoglobin consists of four chains, two α chains and two β chains, each of them has a cyclic heme containing an iron atom. Oxygen is bound to the iron atom and transported by erythrocytes for using by the body.

The hematopoietic stem cells or the preparations thereof obtained according to the method of the present invention may be used to treat a disease selected from the group consisting of: an anemia disease, a blood loss disease, a tumor, or other disease requiring massive blood transfusion for treatment. In particular, it may be used to treat β thalassemia or sickle cell anemia.

In some embodiments, the HSPCs erythroid expansion and differentiation medium comprises: a basal medium, and a composition of growth factors, wherein the composition of growth factors comprises a stem cell growth factor (SCF), interleukin 3 (IL-3) and erythropoietin (EPO).

In some embodiments, the erythroid differentiation and enucleation medium comprises a basal medium, growth factors, and antagonists and/or inhibitors of a progesterone receptor and a glucocorticoid receptor.

When the genetically modified hematopoietic stem cells are cultured by the above method, the hematopoietic stem cells may be differentiated into mature erythrocytes by two steps, compared with the prior art, only 14 days are needed for the in vitro differentiation culture according to the present invention, and the time period is shorter and greatly shortened as compared with a time period of more than 21 days needed in the prior art.

In some embodiments, the growth factors comprise erythropoietin (EPO), and the antagonists and/or inhibitors of a progesterone receptor and a glucocorticoid receptor are any one, or two or more selected from the following compounds (I)-(IV):

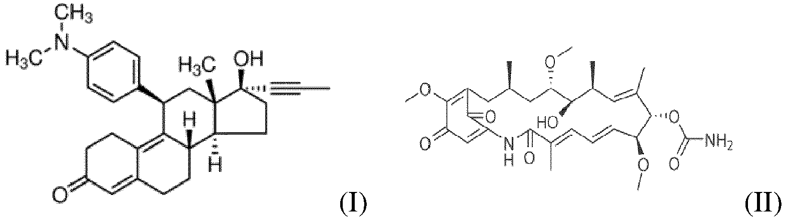

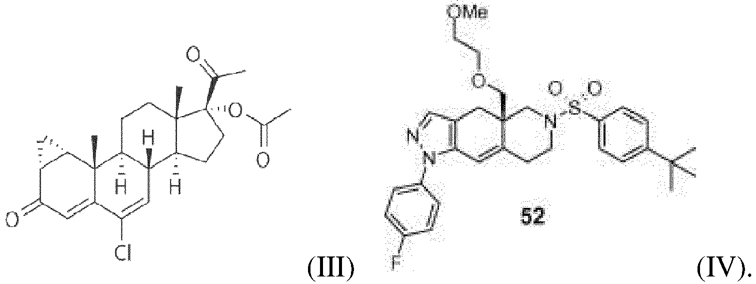

In some embodiments, the HSPCs erythroid expansion and differentiation medium comprises a basal medium, such as STEMSPAN^{™} SFEM II (STEM CELLS TECHNOLOGY Inc.), IMDM (Iscove's Modified Dulbecco's Medium), X-VIVO 15, alpha-MEM, RPMI 1640, and DF12 and the like. As for the growth factors, e.g., if STEMSPAN^{™} SFEM II is used as the basal medium, additional growth factors including 50-200 ng/ml SCF, 10-100 ng/ml IL-3, 1-10 U/ml EPO are needed to add into the medium, wherein the unit of U is defined as: the amount of protein that can convert 1 µmol of substrate in 1 min under specific conditions, i.e., 1 IU=1 µmol/min, at present, the letter "I" of "IU (international unit)" is often omitted in most clinical laboratories at home and abroad, and it is abbreviated as "U". Erythropoietin (EPO) is a glycoprotein mainly secreted by the kidney in response to hypoxia or anemia, and in this embodiment, it promotes the differentiation of hematopoietic stem cells, and usually the time period of its effect is about 7 days.

If a basal medium other than STEMSPAN^{™} SFEM II is adopted as the basal medium, the following ingredients are needed:100X ITS (insulin-transferrin-selenium) (wherein the final concentrations of each substance in the ITS of the medium are as follows: insulin concentration is 0.1 mg/ml; human transferrin, 0.0055 mg/ml; selenium, 6.7^{∗}10⁻⁶ mg/ml) (i.e., mainly including insulin, human transferrin, and selenium); 10-50µg/ml vitamin C; 0.5-5% BSA (bovine serum albumin); growth factors, such as 50-200 ng/ml SCF, 10-100 ng/ml IL-3, and 1-10 U/ml EPO.

Furthermore, those skilled in the art will appreciate that any of the commonly used basal media may be used, for example, basal media commonly used in the art listed as follows: STEMSPAN^{™} SFEM II (purchased from STEM CELL TECHONOLOGIES); and IMDM; DF12; Knockout DMEM; RPMI 1640; Alpha MEM; DMEM, etc. available from Thermo Fisher.

Further, some other ingredients may also be added to the above medium as needed, for example, ITS (i.e., mainly including insulin, human transferrin, and selenium), L-glutamine, vitamin C, and bovine serum albumin. For example, ITS, 2 mM L-glutamine, 10-50 µg/ml vitamin C, and 0.5-5 wt% BSA (bovine serum albumin) may be added into the IMDM medium. In addition, the same concentration of ITS, L-glutamine, vitamin C and bovine serum albumin may also be added into the above DF12. The same concentration of ITS, L-glutamine, vitamin C and bovine serum albumin may be added into the Knockout DMEM; the same concentration of ITS, L-glutamine, vitamin C and bovine serum albumin may also be added into the RPMI 1640; the same concentration of ITS, L-glutamine, vitamin C and bovine serum albumin may also be added into the alpha MEM; the same concentration of ITS, L-glutamine, vitamin C and bovine serum albumin may also be added into the DMEM. Herein, the concentrations of each substance in the ITS in various basal mediums are: insulin concentration is 0.1 mg/ml; human transferrin, 0.0055 mg/ml; and selenium, 6.7×10⁻⁶ mg/ml. In addition, the concentrations of each component of the added ITS may also be adjusted according to actual needs. ITS may be purchased from Thermofisher, and adjusted to the appropriate final concentration for use as needed.

In one embodiment, the erythroid differentiation and enucleation medium comprises a basal medium, growth factors, and antagonists of a progesterone receptor and a glucocorticoid receptor.

In one embodiment, the hematopoietic stem cell erythroid differentiation and enucleation medium comprises a basal medium, such as STEMSPAN^{™} SFEM II (STEM CELLS TECHNOLOGY Inc.), IMDM (Iscove's Modified Dulbecco's Medium), X-VIVO 15, alpha-MEM , RPMI 1640 and DF12, etc. It also contains growth factors, for example, if STEMSPAN^{™} SFEM II is used as the basal medium, additional growth factors needed comprises: 1-10 U/ml EPO, 100-1000 µg/ml human transferrin, and chemical small molecules of 0.5-10 µmol/ml mifepristone.

If a basal medium other than STEMSPAN^{™} SFEM II is used, it is necessary to add, for example, ITS (insulin-transferrin-selenium) (wherein the final concentrations of each substance in the ITS in the medium are: insulin concentration is 0.1 mg/ml; human Transferrin, 0.0055 mg/ml; and selenium, 6.7×10⁻⁶mg/ml) (i.e., mainly including insulin, human transferrin and selenium); vitamin C, 10-50ug/ml; BSA (bovine serum albumin), 0.5-5%; growth factors, such as EPO, 1-10 U/ml; human transferrin, 100-1000 ug/ml; and chemical small molecules such as mifepristone, 0.5-10 µmol/ml.

Furthermore, those skilled in the art will appreciate that any of the commonly used basal media may be used. For example, the basal media commonly used in the art listed as follows: STEMSPAN^{™} SFEM II (purchased from STEM CELL TECHONOLOGIES); IMDM; DF12; Knockout DMEM; RPMI 1640; Alpha MEM; and DMEM, etc., available from Thermo Fisher.

Further, some other ingredients also may be added to the above medium as needed, for example, ITS (i.e., mainly including insulin, human transferrin, and selenium), L-glutamine, vitamin C, and bovine serum albumin may be further added. For example, ITS, plus 2 mM L-glutamine, plus 10-50 µg/ml vitamin C, and 0.5-5 wt% BSA (bovine serum albumin) may be added into the IMDM medium. In addition, the same concentration of ITS, L-glutamine, vitamin C and bovine serum albumin may also be added into the above DF12. The same concentration of ITS, L-glutamine, vitamin C and bovine serum albumin may be added into the Knockout DMEM; the same concentration of ITS, L-glutamine, vitamin C and bovine serum albumin may also be added into the RPMI 1640; the same concentration of ITS, L-glutamine, vitamin C and bovine serum albumin may also be added into the alpha MEM; the same concentration of ITS, L-glutamine, vitamin C and bovine serum albumin may also be added into the DMEM. Herein, the concentrations of each substance in the ITS in various basal mediums are: insulin concentration is 0.1 mg/ml; human transferrin, 0.0055 mg/ml; and selenium, 6.7×10⁻⁶ mg/ml. In addition, the concentrations of each component in the added ITS may also be adjusted according to actual needs. ITS may be purchased from Thermofisher, and adjusted to the appropriate final concentration for use as needed.

Mifepristone used herein is a chemically synthesized small molecule as an antagonist of the progesterone receptor and the glucocorticoid receptor, and it has the following chemical structural formula:

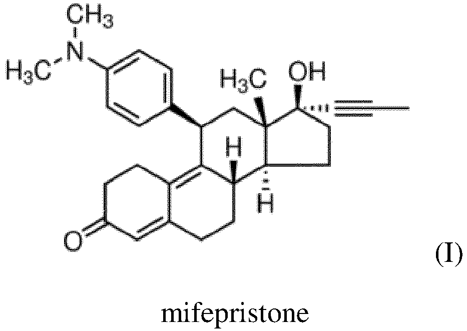

Other antagonists and inhibitors of a progesterone receptor and a glucocorticoid receptor may also be used in the present invention, including cyproterone acetate, geldanamycin, CORT 108297 and the like. The chemical structures are as follows:

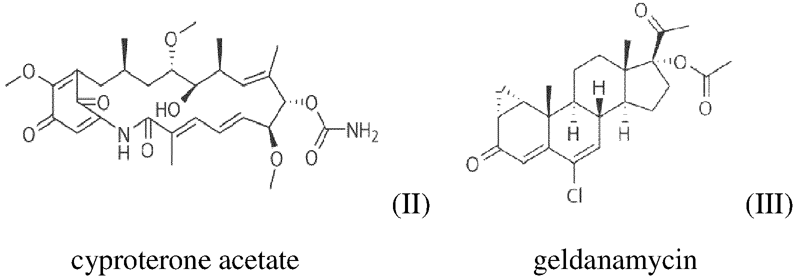

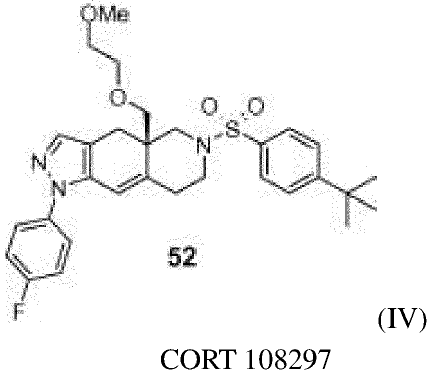

In some embodiments, the mature erythrocytes may be produced by a method comprising the following steps a)-c): a) isolating CD34-positive HSPCs from human umbilical cord blood to perform gene modification by the method of the invention; b) amplifying and differentiating the genetically modified HSPCs for 5-10 days, e.g., 5 days, 6 days, 7 days, 8 days, 9 days, 10 days, to obtain erythroid precursor cells, such as erythroblasts, nucleated erythrocytes, young erythrocytes, and reticulocytes, c) further differentiating the erythroid precursor cells for 7 days to obtain mature erythrocytes.

In some embodiments, the mature erythrocytes be produced by a method comprising the following steps a)-d):
a) isolating CD34-positive HSPCs from human umbilical cord blood by magnetic bead sorting;
b) genetically modifying the CD34-positive HSPCs by the method of the invention;
c) adding additional growth factors into serum-free medium (SFME), and after 5-10 days, e.g., 5 days, 6 days, 7 days, 8 days, 9 days, 10 day of amplification and differentiations, the HSPCs are differentiated into erythrocyte precursor cells, and the medium at this stage is named as HSPCs erythroid expansion and differentiation medium (abbreviated as HEEDM);
d) adding additional growth factors into a serum-free medium (SFME), and after 7 days of differentiation, the mature erythrocytes may be obtained, the medium at this stage is named as HSPCs erythroid differentiation and enucleation medium (abbreviated as HEDEM).

That is, for genetically edited CD34-positive hematopoietic stem cells, the mature erythrocytes may be obtained in two steps by the method of the present invention as claimed, and the time period of the whole process may be 10-18 days, 11-17 days, 12-16 days, 13-15 days, 10-17 days, 10-16 days, 10-15 days, or 10-14 days, such a time period is greatly shorter than the period of at least 21 days in the prior art by using three or four steps.

As described above, the present invention relates to a method for preparing mature erythrocytes or precursor cells thereof being genetically modified to increase the expression of fetal hemoglobin (HbF), which comprises: (a) obtaining the hematopoietic stem cells by the genetic modification method of the present invention; (b) performing hematopoietic stem cell erythroid expansion and differentiation of the genetically modified hematopoietic stem cells by using the above HSPCs erythroid expansion and differentiation medium.

Further, the present invention relates to a method for preparing mature erythrocytes or precursor cells thereof being genetically modified to increase the expression of fetal hemoglobin (HbF), which comprises: (a) obtaining the hematopoietic stem cells by the genetic modification method of the present invention; (b) performing hematopoietic stem cell erythroid expansion and differentiation of the genetically modified hematopoietic stem cells by using the above HSPCs erythroid expansion and differentiation medium; and (c) performing HSPCs erythroid differentiation and enucleation by using the erythroid differentiation and enucleation medium. The present invention also relates to use of the HSPCs erythroid expansion and differentiation medium and/or the erythroid differentiation and enucleation medium used in the above method for amplifying and/or differentiating the hematopoietic stem cells or the precursor cells of mature erythrocytes.

As used herein, "differentiation" refers to a phenomenon in which the structure or function of a cell is specialized during the process of division, proliferation, and growth thereof, that is, the characteristic and function of an organism's cells or tissues are altered to perform a function imparted to the cells or tissues. In general, it refers to the phenomenon in which a relatively simple system is divided into two or more partial systems having different properties.

As used herein, "Ficoll liquid density gradient centrifugation" utilizes the basic principle that the specific gravity of different components in the blood are different, and different cells may be separated in layers through low-speed density gradient centrifugation. The density of erythrocytes and granulocytes is greater than that of the stratified liquid, and when erythrocytes encounter ficoll liquid they will quickly aggregate into a string arrangement and accumulate at the bottom of the tube. Only the mononuclear cells having a density equivalent to that of the stratified liquid are enriched between the plasma layer and the stratified liquid, i.e., the white film layer; the hematopoietic stem cells are present in this layer and they may be obtained by subsequent magnetic bead sorting. In the present invention, the mononuclear cells are obtained by using commercially available lymphocyte separation tubes.

As used herein, "mature erythrocytes" refers to the most abundant type of cells in blood, and they have the function of carrying nutrients such as oxygen, amino acids, and carbon dioxide, and the like. Mature erythrocytes have no mitochondria and nuclei.

As used herein, "interleukin 3 (IL-3)" refers to a cytokine produced by activated CD4- and CD8-positive T lymphocytes, and its primary biological function is to participate in regulating the proliferation and differentiation of the hematopoietic stem cells in the bone marrow..

As used herein, "erythropoietin (EPO)" refers to a growth factor produced by erythroblasts, i.e., erythroid precursor cells. In an adult, there are glycoproteins secreted by the interstitial cells surrounding the renal tubules in renal cortex and the liver. EPO can stimulate the differentiation of hematopoietic stem cells into erythrocytes.

In some embodiments, the stem cell factors (SCFs) comprise mast cell growth factor (MGF), kit ligand (KL), and steel factor (SLF).

As used herein, "HSPCs erythroid expansion and differentiation medium" refers to a culturing system for promoting the massive expansion of HSPCs and their further differentiation into erythroid progenitor cells. In the present invention, the medium contains two main components, a basic medium and a growth factor additive. The basal medium is a serum-free system, either STEMSPAN^{™} SFEM II (STEM CELLS TECHNOLOGY Inc.) or IMDM (Iscove's Modified Dulbecco's Medium), plus ITS (Thermofisher), L-gulutamin (Thermofisher), vitamin C, and bovine serum albumin. The growth factor additive is a combination of different concentrations of IL-3, SCF, and EPO.

As used herein, "HSPCs erythroid differentiation and enucleation medium" refers to a medium that promotes the further expansion and differentiation of erythroid progenitor cells into enucleated erythrocytes. In the present invention, the medium comprises two main components, namely a basic medium, and additives of growth factors and chemical small molecules. The basal medium is a serum-free system, either STEMSPAN^{™} SFEM II (STEM CELLS TECHNOLOGY Inc.), or IMDM (Iscove's Modified Dulbecco's Medium), plus ITS (Thermofisher), L-gulutamin (Thermofisher), vitamin C, and bovine serum albumin. Additives of growth factors and chemical small molecules include EPO, human transferrin, and the chemical small molecule of mifepristone.

In some embodiments, the hematopoietic stem cells are sorted by magnetic beads. For example, the cells are specifically labeled with super paramagnetic MACS microbeads, and after magnetic labeling, making the cells to pass through a sorting column placed in a strong and stable magnetic field. The matrix in the sorting column creates a high gradient magnetic field.

The magnetically labeled cells are retained in the column while the unlabeled cells are discharged. When the sorting column is moved out of the magnetic field, the magnetically labeled cells retained in the column may be eluted. Thus, both the labeled and unlabeled cell components may be completely obtained.

In some specific embodiments of the present invention, through utilizing the sgRNA the hematopoietic stem cells derived from three different umbilical cord blood are genetically modified by the genetic modification methods of the invention, and efficient gene editing may be achieved with an efficiency of at least 40% or more, for example, 50% or more, 60% or more, 70% or more, 80% or more, or 90% or more. For example, as for the preferred sgRNA, the average efficiency of gene editing even reaches 80%.

In some embodiments of the present invention, the hematopoietic stem cells obtained by the genetic editing method of the present invention are transplanted into a mouse. The proportion of human hCD45 expression in the mouse continuously increases after the transplantation of the genetically edited hematopoietic stem cells, as compared with that of the transplantation of the hematopoietic stem cells not being genetically modified. In peripheral blood samples, the proportion of hCD45 expression increases from 20% at week 6 to 60% at week 16; the proportion even reaches 90% in bone marrow and 70% in spleen at week 16; indicating that the genetically modified hematopoietic stem cells may be rapidly and efficiently implanted into the hematopoietic system of the mouse model, and the in vivo differentiation function of the cells is normal.

In some embodiments of the present invention, the umbilical cord blood-derived hematopoietic stem cells are modified by the gene editing method of the present invention, and then they are differentiated by using the "two-step" differentiation method of the present invention, that is, the HSPCs erythroid expansion and differentiation medium is used for expansion and differentiation, and then the HSPCs erythroid differentiation and enucleation medium is used for further differentiation. The detection results show that, the expression of hemoglobin (HBG) in differentiated erythrocytes is increased by 20%-90%, and even by 100% as compared with that in the original erythrocytes, that is, increased by about 1 time (2 times of the expression of the original erythrocytes); the expression of fetal hemoglobin is also increased by 20%-90%, even increased up to 100% as compared with that of the original erythrocytes, that is, about 1 times higher (2 times of the expression of the original erythrocytes); even up to 200%, that is, about 2 times higher (3 times of the original); even up to 300%, that is, about 3 times higher (4 times of the original); even up to 400%, that is, about 4 times higher (5 times of the original) ); even up to 500% or more, that is, about 5 times higher or more (6 or more times of the original), and the like.

### Examples

Hereinafter, the present invention will be further described with reference to the following examples. It is obvious to those skilled in the art that these examples are for illustrative purposes. The scope of the invention is to be defined by the appended claims.

### Example 1: Efficient gene editing of umbilical cord blood-derived CD34-positive hematopoietic stem cells

### 1-1: Testing electroporation conditions by using K562 cells

Due to the limited source of hematopoietic stem cells and the high cost of each single isolation, the cancer cell line K562 (purchased from ATCC organization, website: https: //www.atcc.org) is selected as a model cell line for testing electroporation conditions in this example.

Particularly, the specific steps for implementation are described below.

The first experiments: transfecting 5×10⁵ K562 cells with 5µg of GFP mRNA (the sequence of SEQ ID NO: 1) by BTX830 electroporator under the conditions of 250 V, 1 ms; 360 V, 1 ms; 400 V, 1 ms; and 500 V, 1 ms respectively. 4 days after the electroporation, GFP expression and 7-AAD expression are determined by flow cytometry, wherein GFP represents electroporation efficiency, and 7-AAD represents the growth state, i.e. viability of the cells after electroporation.
SEQ ID NO:1: sequence information of GFP mRNA:

atgagtaaaggagaagaacttttcactggagttgtcccaattcttgttgaattagatggtgatgttaatgggcacaaattttctgt
cagtggagagggtgaaggtgatgcaacatacggaaaacttacccttaaatttatttgcactactggaaaactacctgttccat
ggccaacacttgtcactactttctcttatggtgttcaatgcttttcaagatacccagatcatatgaaacggcatgactttttcaag
agtgccatgcccgaaggttatgtacaggaaagaactatatttttcaaagatgacgggaactacaagacacgtgctgaagtc
aagtttgaaggtgatacccttgttaatagaatcgagttaaaaggtattgattttaaagaagatggaaacattcttggacacaaa

ttggaatacaactataactcacacaatgtatacatcatggcagacaaacaaaagaatggaatcaaagttaacttcaaaattag
acacaacattgaagatggaagcgttcaactagcagaccattatcaacaaaatactccaattggcgatggccctgtccttttac
cagacaaccattacctgtccacacaatctgccctttcgaaagatcccaacgaaaagagagaccacatggtccttcttgagttt
gtaacagctgctgggattacacatggcatggatgaactatacaaatag

The second experiments: transfecting 5×10⁵ K562 cells with 5µg of the above GFP mRNA by BTX830 electroporator under the conditions of 250 V, 1 ms; 250 V, 2 ms; 300 V, 0.5 ms; 300 V, 1 ms; 360 V, 0.5 ms; and 360 V; 1 ms respectively. 4 days after the electroporation, GFP expression and 7-AAD expression are determined by flow cytometry, wherein GFP represents electroporation efficiency, and 7-AAD represents the growth state, i.e. viability of the cells after electroporation.

The third experiments: transfecting 5×10⁵ K562 cells with 5µg of the above GFP mRNA by BTX830 electroporator under the conditions of 250 V, 1 ms; 250 V, 1 ms; 300 V, 1 ms; and 300 V, 1 ms respectively. 4 days after the electroporation, GFP expression and 7-AAD expression are determined by flow cytometry, wherein GFP represents electroporation efficiency, and 7-AAD represents the growth state, i.e. viability of the cells after electroporation. The results are shown in FIGs. 2 and 3.

Among them, FIG. 2 shows the fluorescence photomicrographs obtained 4 days after transfecting with GFP under the optimal electroporation conditions of the multi-batch experiments on cancer cell line K562. "V" refers to the pulse voltage, and "ms" refers to the pulse time.

FIG. 3 is a graph showing flow cytometry analysis of 7-AAD and statistical analysis of GFP expression obtained 4 days after transfecting with GFP under the optimal electroporation conditions of the multi-batch experiments on cancer cell line K562. 7-AAD negative in flow cytometry analysis indicates cell viability. 7-AAD (7-amino-actinomycin D) is a nucleic acid dye that does not pass through the normal plasma membrane, while the permeability of plasma membrane to 7-AAD increases gradually during the progresses of cell apoptosis and death. 7-AAD emits bright red fluorescence when it is excited by appropriate wavelength. 7-AAD negative cells possess normal viability; GFP efficiency indicates electroporation efficiency. Herein, taking "250-1" as an example, it represents a voltage of 250 V, and a pulse time of 1 ms.

The data of these experiments shows that, under the electroporation conditions of "300V, 1ms", the comprehensive index of electroporation efficiency and cell viability is the highest for cancer cell line K562, and they will be used as the electroporation conditions for subsequent experiments.

### 1-2. Transfecting the hematopoietic stem cells with GFP mRNA under the electroporation conditions of 300 v, 1 ms

5×10⁵ hematopoietic stem cells (purchased from ALLCELLs Biotechnology (Shanghai) Co., Ltd., www.allcells.com) are transfected with the above GFP mRNA by electroporation under the electroporation conditions of "300V, 1 ms" which produce the highest efficiency and viability in the previous step. GFP expression and 7-AAD expression are detected 4 days later. The results are shown in FIGs. 4 and 5.

FIG. 4 shows fluorescence photomicrographs obtained 4 days after transfecting the hematopoietic stem cells with the above GFP mRNA by electroporation under the electroporation conditions of 300V, 1 ms, and the four fields included are the bright field, green channel, red channel, and the overlay of the bright field and the green channel.

FIG. 5 is a flow cytometric analysis of GPF and CD34 protein expression, 4 days after transfecting the hematopoietic stem cells with GFP mRNA by electroporation under the electroporation conditions of 300 V, 1 ms. In FIG. 5, the control group is the hematopoietic stem cells not being transfected with GFP mRNA, and in the flow cytometric analysis CD34 antibodies are not stained.

It can be seen from the results of FIGs. 4 and 5 that, the data of these experiments shows the proportion of GFP expression in the umbilical cord blood-derived hematopoietic stem cells is high under the electroporation conditions of "300 V, 1 ms".

### 1-3: Gene editing of BCL11A locus in the cord blood-derived hematopoietic stem cells by electroporation with CRISPR/Cas9

A. Multiple sgRNAs for locus BCL11A (+58) are designed by using the "CRISPR RGEN TOOLS" software, and the chemically modified sgRNAs (the information shown in FIGs. 6, 7 and 8) are synthesized for the locus 58K of BCL11A enhancer (the sequence of the targeted locus 58K is shown in SEQ ID NO: 2).
SEQ ID NO: 2: the 150-bp sequence at the locus 58K of BCL11A enhancer: 
ctgccagtcctcttctaccccacccacgcccccaccctaatcagaggccaaacccttcctggagcctgtgataaaagca
actgttagcttgcactagactagcttcaaagttgtattgaccctggtgtgttatgtctaagagtagatgcc
SEQ ID NO: 3: referred to as BCL11A Enhancer-1 sgRNA (sometimes abbreviated as Enhancer-1):
   cacaggctccaggaagggtt
SEQ ID NO: 4: referred to as BCL11A Enhancer-2 sgRNA (sometimes abbreviated as Enhancer-2):
   atcagaggccaaacccttcc
SEQ ID NO: 5: referred to as BCL11A Enhancer-3 sgRNA (sometimes abbreviated as Enhancer-3):
   ctaacagttgcttttatcac
SEQ ID NO: 6: referred to as BCL11A Enhancer-4 sgRNA (sometimes abbreviated as Enhancer-4):
   ttgcttttatcacaggctcc
SEQ ID NO: 7: referred to as BCL11A Enhancer-5 sgRNA (sometimes abbreviated as Enhancer-5):
   ttttatcacaggctccagga
SEQ ID NO: 8: referred to as BCL11A Enhancer-6 sgRNA (sometimes abbreviated as Enhancer-6):
   tttatcacaggctccaggaa
SEQ ID NO: 9: referred to as BCL11A Enhancer-7 sgRNA (sometimes abbreviated as Enhancer-7):
   tgggtggggtagaagaggac
SEQ ID NO: 10: referred to as BCL11A Enhancer-8 sgRNA (sometimes abbreviated as Enhancer-8):
   gggcgtgggtggggtagaag
SEQ ID NO: 11: referred to as BCL11A Enhancer-9 sgRNA (sometimes abbreviated as Enhancer-9):
   ttagggtgggggcgtgggtg
SEQ ID NO: 12: referred to as BCL11A Enhancer-10 sgRNA (sometimes abbreviated as Enhancer-10):
   attagggtgggggcgtgggt
SEQ ID NO: 13: referred to as BCL11A Enhancer-11 sgRNA (sometimes abbreviated as Enhancer-11):
   gattagggtgggggcgtggg
SEQ ID NO: 14: referred to as BCL11A Enhancer-12 sgRNA (sometimes abbreviated as Enhancer-12):
   tctgattagggtgggggcgt
SEQ ID NO: 15: referred to as BCL11A Enhancer-13 sgRNA (sometimes abbreviated as Enhancer-13):
   ctctgattagggtgggggcg
SEQ ID NO: 16: referred to as BCL11A Enhancer-14 sgRNA (sometimes abbreviated as Enhancer-14):
   cacgcccccaccctaatcag
SEQ ID NO: 17: referred to as BCL11A Enhancer-15 sgRNA (sometimes abbreviated as Enhancer-15):
   ttggcctctgattagggtgg
SEQ ID NO: 18: referred to as BCL11A Enhancer-16 sgRNA (sometimes abbreviated as Enhancer-16):
   tttggcctctgattagggtg
SEQ ID NO: 19: referred to as BCL11A Enhancer-17 sgRNA (sometimes abbreviated as Enhancer-17):
   gtttggcctctgattagggt
SEQ ID NO: 20: referred to as BCL11A Enhancer-18 sgRNA (sometimes abbreviated as Enhancer-18):
   ggtttggcctctgattaggg
SEQ ID NO: 21: referred to as BCL11A Enhancer-19 sgRNA (sometimes abbreviated as Enhancer-19):
   aagggtttggcctctgatta
SEQ ID NO: 22: referred to as BCL11A Enhancer-20 sgRNA (sometimes abbreviated as Enhancer-20):
   gaagggtttggcctctgatt
SEQ ID NO: 23: referred to as BCL11A Enhancer-21 sgRNA (sometimes abbreviated as Enhancer-21):
   actcttagacataacacacc
SEQ ID NO: 24: referred to as BCL11A Enhancer-22 sgRNA (sometimes abbreviated as Enhancer-22):
   cttcaaagttgtattgaccc
SEQ ID NO: 25: referred to as BCL11A Enhancer-23 sgRNA (sometimes abbreviated as Enhancer-23):
   ctcttagacataacacacca

As described above, the above 23 sgRNAs are designed for the 150-bp sequence at the locus 58K of BCL11A enhancer.

B. The electroporation conditions of "300 V, 1 ms" are used, and the inventors synthesized Cas9 mRNA (the sequence of SEQ ID NO: 26) and the 23 chemically modified sgRNAs which are designed as described above, wherein the chemical modification of the 23 sgRNAs is 2'-O-methyl modification and internucleotide 3'-thio modification of the first three bases at the 5' end and the last three bases at the 3' end of the sgRNA. As shown in the formulas below, the chemically modified sgRNA is shown on the left, and unmodified sgRNA is shown on the right.

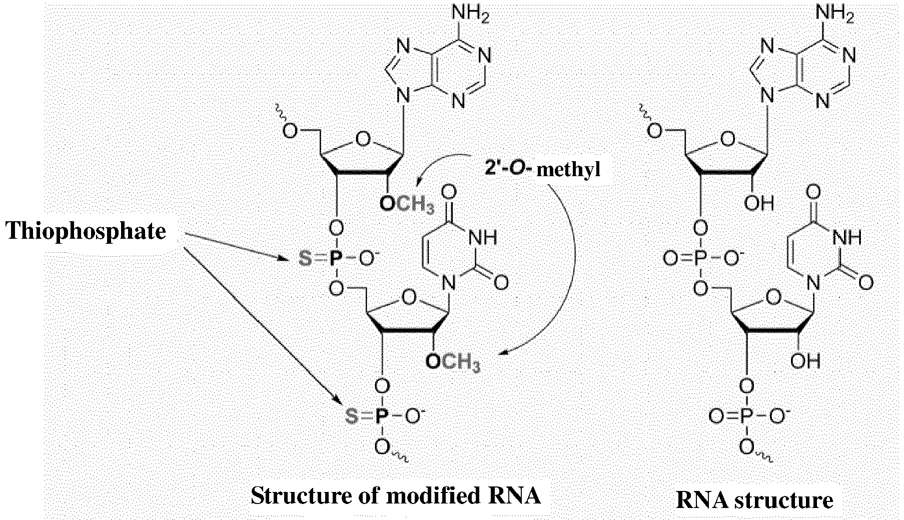

The cord blood-derived CD34-positive hematopoietic stem cells (purchased from ALLCELLS Biotechnology (Shanghai) Co., Ltd., www.allcells.com) are transfected with the above 23 chemically modified sgRNAs by electroporation under the above-identified electroporation conditions, analyzing the Indels efficiency by TIDE software 4 says later. The results are shown in FIG. 9. Also, in this example, as a comparison, the unmodified sgRNA is also used for transfection by electroporation in the same method, but the unmodified sgRNA produces an Indels efficiency of only 2.7%. The chemically modified sgRNAs are used in the following examples.

FIG. 9 shows that the CD34-positive hematopoietic stem cells are transfected with Cas9 mRNA and the 23 sgRNAs by electroporation. The genome of the CD34-positive hematopoietic stem cells is extracted after 4 days, and a fragment covering about 450 bp at the left and right side of the cleavage site of the sgRNA (with a total of 903 bp in length) is selected for amplification. The primer sequences for amplification are as follows:
Forward primer: cacctcagcagaaacaaagttatc (SEQ ID NO: 29)
Reverse primer: gggaagctccaaactctcaa (SEQ ID NO: 30)
Cleavage site selection: in the case of Enhancer-3, 5'-ctaaacagttg cttttatcac-3' (SEQ ID NO: 5), the cleavage site is at the right site of the 3' end (Cong L, et al. Science. 2013).

Sanger sequencing is performed to amplify the above fragment by using the upstream and downstream primer sequences as shown in SEQ ID NO: 29 and SEQ ID NO: 30. Based on the sequencing results,statistic analysis of the resulting Indels efficiency is performed by using TIDE software, wherein the TIDE software is an on-line software for Indels efficiency analysis. The efficiency of Indels for producing double-peak mutation may be analyzed according to the first-generation sequencing results by referring to Tide. deskgen. com.

The results show that all the 23 sgRNAs synthesized in this example may be used to successfully perform gene editing of the hematopoietic stem cells, and Indels may be effectively produced with an efficiency of at least 10%. The most efficient one of them is Enhancer-2.
SEQ ID NO: 26: the sequence of Cas9 mRNA 
gacaagaagtacagcatcggcctggacatcggcaccaactctgtgggctgggccgtgatcaccgacgagtacaaggtg
cccagcaagaaattcaaggtgctgggcaacaccgaccggcacagcatcaagaagaacctgatcggagccctgctgttcg
acagcggcgaaacagccgaggccacccggctgaagagaaccgccagaagaagatacaccagacggaagaaccgga
tctgctatctgcaagagatcttcagcaacgagatggccaaggtggacgacagcttcttccacagactggaagagtccttcct
ggtggaagaggataagaagcacgagcggcaccccatcttcggcaacatcgtggacgaggtggcctaccacgagaagt
accccaccatctaccacctgagaaagaaactggtggacagcaccgacaaggccgacctgcggctgatctatctggccct
ggcccacatgatcaagttccggggccacttcctgatcgagggcgacctgaaccccgacaacagcgacgtggacaagct
gttcatccagctggtgcagacctacaaccagctgttcgaggaaaaccccatcaacgccagcggcgtggacgccaaggcc
atcctgtctgccagactgagcaagagcagacggctggaaaatctgatcgcccagctgcccggcgagaagaagaatggc
ctgttcggcaacctgattgccctgagcctgggcctgacccccaacttcaagagcaacttcgacctggccgaggatgccaa
actgcagctgagcaaggacacctacgacgacgacctggacaacctgctggcccagatcggcgaccagtacgccgacct
gtttctggccgccaagaacctgtccgacgccatcctgctgagcgacatcctgagagtgaacaccgagatcaccaaggcc
cccctgagcgcctctatgatcaagagatacgacgagcaccaccaggacctgaccctgctgaaagctctcgtgcggcagc
agctgcctgagaagtacaaagagattttcttcgaccagagcaagaacggctacgccggctacattgacggcggagccag
ccaggaagagttctacaagttcatcaagcccatcctggaaaagatggacggcaccgaggaactgctcgtgaagctgaac
agagaggacctgctgcggaagcagcggaccttcgacaacggcagcatcccccaccagatccacctgggagagctgca
cgccattctgcggcggcaggaagatttttacccattcctgaaggacaaccgggaaaagatcgagaagatcctgaccttcc
 
gcatcccctactacgtgggccctctggccaggggaaacagcagattcgcctggatgaccagaaagagcgaggaaacca
tcaccccctggaacttcgaggaagtggtggacaagggcgcttccgcccagagcttcatcgagcggatgaccaacttcgat
aagaacctgcccaacgagaaggtgctgcccaagcacagcctgctgtacgagtacttcaccgtgtataacgagctgaccaa
agtgaaatacgtgaccgagggaatgagaaagcccgccttcctgagcggcgagcagaaaaaggccatcgtggacctgct
gttcaagaccaaccggaaagtgaccgtgaagcagctgaaagaggactacttcaagaaaatcgagtgcttcgactccgtg
gaaatctccggcgtggaagatcggttcaacgcctccctgggcacataccacgatctgctgaaaattatcaaggacaagga
cttcctggacaatgaggaaaacgaggacattctggaagatatcgtgctgaccctgacactgtttgaggacagagagatgat
cgaggaacggctgaaaacctatgcccacctgttcgacgacaaagtgatgaagcagctgaagcggcggagatacaccgg
ctggggcaggctgagccggaagctgatcaacggcatccgggacaagcagtccggcaagacaatcctggatttcctgaa
gtccgacggcttcgccaacagaaacttcatgcagctgatccacgacgacagcctgacctttaaagaggacatccagaaag
cccaggtgtccggccagggcgatagcctgcacgagcacattgccaatctggccggcagccccgccattaagaagggca
tcctgcagacagtgaaggtggtggacgagctcgtgaaagtgatgggccggcacaagcccgagaacatcgtgatcgaaa
tggccagagagaaccagaccacccagaagggacagaagaacagccgcgagagaatgaagcggatcgaagagggca
tcaaagagctgggcagccagatcctgaaagaacaccccgtggaaaacacccagctgcagaacgagaagctgtacctgt
actacctgcagaatgggcgggatatgtacgtggaccaggaactggacatcaaccggctgtccgactacgatgtggaccat
atcgtgcctcagagctttctgaaggacgactccatcgacaacaaggtgctgaccagaagcgacaagaaccggggcaag
agcgacaacgtgccctccgaagaggtcgtgaagaagatgaagaactactggcggcagctgctgaacgccaagctgatt
acccagagaaagttcgacaatctgaccaaggccgagagaggcggcctgagcgaactggataaggccggcttcatcaag
agacagctggtggaaacccggcagatcacaaagcacgtggcacagatcctggactcccggatgaacactaagtacgac
gagaatgacaagctgatccgggaagtgaaagtgatcaccctgaagtccaagctggtgtccgatttccggaaggatttcca
gttttacaaagtgcgcgagatcaacaactaccaccacgcccacgacgcctacctgaacgccgtcgtgggaaccgccctg
atcaaaaagtaccctaagctggaaagcgagttcgtgtacggcgactacaaggtgtacgacgtgcggaagatgatcgcca
agagcgagcaggaaatcggcaaggctaccgccaagtacttcttctacagcaacatcatgaactttttcaagaccgagatta
ccctggccaacggcgagatccggaagcggcctctgatcgagacaaacggcgaaaccggggagatcgtgtgggataag
ggccgggattttgccaccgtgcggaaagtgctgagcatgccccaagtgaatatcgtgaaaaagaccgaggtgcagacag
gcggcttcagcaaagagtctatcctgcccaagaggaacagcgataagctgatcgccagaaagaaggactgggaccctaa
gaagtacggcggcttcgacagccccaccgtggcctattctgtgctggtggtggccaaagtggaaaagggcaagtccaa
gaaactgaagagtgtgaaagagctgctggggatcaccatcatggaaagaagcagcttcgagaagaatcccatcgactttc
tggaagccaagggctacaaagaagtgaaaaaggacctgatcatcaagctgcctaagtactccctgttcgagctggaaaac
ggccggaagagaatgctggcctctgccggcgaactgcagaagggaaacgaactggccctgccctccaaatatgtgaac
 
ttcctgtacctggccagccactatgagaagctgaagggctcccccgaggataatgagcagaaacagctgtttgtggaaca
gcacaagcactacctggacgagatcatcgagcagatcagcgagttctccaagagagtgatcctggccgacgctaatctg
gacaaagtgctgtccgcctacaacaagcaccgggataagcccatcagagagcaggccgagaatatcatccacctgtttac
cctgaccaatctgggagcccctgccgccttcaagtactttgacaccaccatcgaccggaagaggtacaccagcaccaaa
gaggtgctggacgccaccctgatccaccagagcatcaccggcctgtacgagacacggatcgacctgtctcagctggga
ggcgac
SEQ ID NO: 27, sequencing primer
   Cacctcagcagaaacaaagttatc
SEQ ID NO: 28, sequencing primer
   gggaagctccaaactctcaa

C. According to the above experimental results, Enhancer-2, Enhancer-3, Enhancer-4, Enhancer-5 and Enhancer-6 with relatively high gene editing efficiency are selected. The umbilical cord blood-derived hematopoietic stem cells are transfected with Cas9 mRNA and Enhancer-2, Enhancer-3, Enhancer-4, Enhancer-5 or Enhancer-6 by electroporation under the electroporation conditions of 300V, 1ms; and the Indels efficiency is determined after 4 days, and the results are shown in FIG. 10. FIG. 10 shows the statistical analysis of the Indels efficiency by using the same method as above and the TIDE software, after transfecting the CD34-positive hematopoietic stem cells derived from 3 different cord blood sources with Cas9 mRNA and BCL11A Enhancer-2 sgRNA, Enhancer-3 sgRNA, Enhancer-4 sgRNA, Enhancer-5 sgRNA, and Enhancer-6 sgRNA respectively by electroporation 4 days later.

The results in FIG. 10 shows that, 5 sgRNAs achieve efficient gene editing in hematopoietic stem cells derived from 3 different cord blood sources with the efficiencies of at least 40% or more. Enhancer-2 sgRNA has the highest efficiency, the average gene editing efficiency of Enhancer-2 sgRNA is 80%, and it is significantly higher than that of Enhancer-3, Enhancer-4, Enhancer-5, and Enhancer-6 sgRNA. Meanwhile, the gene editing efficiency in hematopoietic stem cells is higher than that reported in the existing literatures (Xu, et al. Molecular Therapy. 2017; DeWitt, et al. Sci Transl Med. 2016).

Based on this, Enhancer-2 sgRNA is used as a target in the subsequent experiments.

### Example 2: In vitro colony-formation of the genetically modified hematopoietic stem cells derived from cord blood

This experiment involves the detection of colony forming unit (CFU) of the genetically modified hematopoietic stem cells derived from cord blood.

The hematopoietic stem cells are transfected with Cas9 mRNA and Enhancer-2 under the electroporation conditions of 300 V, 1 ms. 800-1000 cells are resuspended in 1 ml of the mixture of H4434 (available from STEM CELLS TECHNOLOGY, Canada), IMDM (available from Thermo Fisher) and FBS (available from Thermo Fisher). The number of the formed colonies with different morphologies such as CFU-M, BFU-E, CFU-E, CFU-G, CFU-GM are observed under microscope after 14 days, and the results are shown in FIG. 11. Among them, FIG. 11 shows the number of colonies for different blood systems, which is obtained by transfecting CD34-positive hematopoietic stem cells derived from cord blood with Cas9 mRNA and BCL11A enhancer-2 sgRNA by electroporation, performing *in vitro* colony-forming units assay (CFU assay) 2 days later, and then counting the number of colonies for different blood systems 14 days later; wherein BFU-E, CFU-M, CFU-GM, CFU-E, CFU-G, and CFU-MM represent the cell colonies of different lineages, such as erythroid, myeloid, and lymphoid lineage, etc. in blood system, and wherein Mock represents cells not being genetically modified.

According to the data shown in example 2, compared with that of the hematopoietic stem cells not being genetically modified, the *in vitro* differentiation function of the genetically modified cells is normal, and the cells are differentiated into colonies of different lineages of blood system.

### Example 3: The reconstruction of hematopoietic system in mouse model with genetically modified hematopoietic stem cells derived from cord blood

The cord blood-derived hematopoietic stem cells are transfected with Cas9 mRNA and Enhancer-2 by electroporation under the electroporation conditions of 300 V, 1 ms; transplanting into an irradiated NPG immunodeficient mouse model (purchased from Beijing Vitalstar Biotechnology, Inc.). The expression of human CD45 and mouse CD45 in peripheral blood is detected 6 weeks, 8 weeks, 10 weeks, 12 weeks and 16 weeks after the transplantation, while the expression of human CD45 and mouse CD45 in bone marrow and spleen is detected 16 weeks after transplantation; and the results are shown in FIGs. 12 and 14. The method for transplantation into the mice comprises the following steps: removing bone marrow from the mouse model by 1.0 Gy irradiation twenty-four hours prior to the cell transplantation; then resuspending 1.0×10⁶ cells in 20 µL of 0.9% saline and injecting through the tail vein of the mice which are subsequently raised in a clean room.

The results of FIGs. 12 and 14 show that, after transplantation into a mouse model, as compared with the genetically unmodified hematopoietic stem cells, the proportion of human hCD45 expression in the genetically modified hematopoietic stem cells continues to increase over time, and it increases from 20% at week 6 to 60% at week 16 in the peripheral blood samples, and even reaches 90% in bone marrow and 70% in spleen at week 16, indicating that the genetically modified hematopoietic stem cells may be transplanted quickly and efficiently into the hematopoietic system of a mouse model, and the *in vivo* differentiation function of the cells is normal. The reports of the existing literatures show that, the proportion of CD45 expression in peripheral blood is 1-10% after 6 weeks, the proportion of CD45 expression in peripheral blood is 20-40% after 16 weeks, and the proportion of CD45 expression in bone marrow is about 50%. Thus, the results of the animal experiments in the invention are obviously superior to those in the transplantation experiments reported in the prior art (Xu, et al., Molecular Therapy. 2017; DeWitt, et al., Sci Transl Med. 2016; Mettananda, et al., Nature Communications. 2017).

Meanwhile, in mice transplanted with genetically modified cells, the expression of cell membrane proteins such as human CD3, CD4, CD8, CD33, CD19, CD56 is detected after 16 weeks, as shown in FIGs. 13, 14 and 15. The results show that the genetically modified cells express these proteins normally, indicating that the cells may differentiate into T cells, B cells, macrophages and other cells of the blood system, and enable to efficiently repopulate the hematopoietic system of a mouse model. Compared with the results reported in the literatures, firstly, we test six cell membrane surface proteins including CD3, CD4, CD8, CD33, CD19 and CD56, to determine the expression of T cells, myeloid cells, B cells and NK cells in the blood of mice, and evaluate the capability of the transplanted human hematopoietic stem cells for repopulating the hematopoietic system of mice more accurately, while in the existing literatures, only proteins CD3, CD19, CD56, CD33 are tested; secondly, we test the expression profiles of the above six proteins in three important blood system related tissues including peripheral blood, bone marrow, and spleen, to evaluate the capacity of repopulating the hematopoietic system of the mouse more comprehensively; while in the existing literatures, only the bone marrow is taken as the test tissue (Chang, et al. Methods & Clinical Development. 2017; deWitt, et al. Sci Transl Med. 2016; Mettananda, et al. Nature Communications. 2017).

In addition, the genetically modified cells are capable of rapidly and efficiently repopulating the hematopoietic system of a mouse model. The result shown in FIG. 16 is used for judging whether the cells for repopulating a mouse model are genetically modified. Genomes of the cells before transplantation, and genomes of the peripheral blood, bone marrow and spleen 16 weeks after transplantation are extracted, amplifying the target fragments and performing Sanger sequencing. The Indels efficiency is determined by TIDE analysis. The results show that all the human cells in the peripheral blood, bone marrow and spleen are genetically modified 16 weeks after transplantation, and the Indels efficiency is from 50% to 70%, and similar to that of the cells before transplantation.

### Example 4: The erythroid differentiation of the genetically modified hematopoietic stem cells derived from cord blood and the Detection of γ globin and fetal hemoglobin expression

### 4-1. Differentiation of erythrocytes

The cord blood-derived hematopoietic stem cells are transfected with Cas9 mRNA and Enhancer-2 by electroporation under the electroporation conditions of 300 v, 1 ms, differentiating the cells by using the "two-step" differentiation protocol described below.

In the two-step method, firstly the HSPCs erythroid expansion and differentiation medium is used for differentiation, then the HSPCs erythroid differentiation and enucleation medium is used for differentiation.

The basal medium of the HSPCs erythroid expansion and differentiation culture medium is StemSpan^{™} SFEM II, including the growth factors of 50-200ng/ml SCF, 10-100ng/ml IL-3, and 1-10U EPO/ml. The culture conditions: the hematopoietic stem cells are cultured with a density of 1.0×10⁵ cells/ml in the erythroid expansion and differentiation medium for 7 days.

The basal medium of HSPCs erythroid differentiation and enucleation medium is StemSpan^{™} SFEM II including the growth factors of 1-10 U EPO, 100-1000 µg/ml human transferrin, 0.5-10 µm the small molecule of mifepristone. The cells cultured in the previous step with a density of 1.0×10⁶ cells/ml are differentiated in the HSPCs erythroid differentiation and enucleation medium for 5 days.

Then, the expression of CD71 and CD235a is detected, as shown in FIG. 17. In the experimental group and the control group, the cells are differentiated into erythrocytes efficiently, and the proportion of CD71 and CD235a expression is 90% or more.

### 4-2. Detection of the expression of γ globin and fetal hemoglobin

mRNA of the cells differentiated from the above erythrocytes is extracted and reverse-transcribed into cDNA. The expression of BCL11A, HBB, HBG and other genes is detected by quantitative fluorescence PCR, as shown in FIG. 18. The results show that in cells with knockout of the BCL11A enhancer locus, the expression of BCL11A gene is down-regulated by 1-fold, and the expression of HBG is up-regulated by 1-fold.

Fetal hemoglobin expression in cells after erythroid differentiation is detected, as shown in FIG. 19. Flow cytometry analysis shows that in cells with knockout of BCL11A enhancer locus, fetal hemoglobin expression is increased approximately 1-fold.

### Example 5: Gene editing of the BCL11A enhancer locus in the hematopoietic stem cells derived from β thalassemia patients

### 5-1. Isolation of the hematopoietic stem cells from peripheral blood of patients with β thalassemia

CD34-positive hematopoietic stem cells are obtained by conventional magnetic bead sorting. The results are shown in FIG. 20.

### 5-2. Transfecting the hematopoietic stem cells derived from the peripheral blood of β thalassemia patients by electroporation

The peripheral blood-derived hematopoietic stem cells from patients with β thalassemia are transfected with Cas9 mRNA and Enhancer-2, Enhancer-3, Enhancer-4, Enhancer-5, or Enhancer-6 by electroporation under the electroporation conditions of 300 V, 1 ms. Indels efficiency is detected 4 days later, as shown in FIG. 21. The results show that efficient gene editing is achieved in peripheral blood-derived hematopoietic stem cells from 3 different patients by using each of the 5 sgRNAs, wherein Enhancer-2 has the highest efficiency of at least 70% or more.

### Example 6: The erythroid differentiation of the genetically modified hematopoietic stem cells derived from peripheral blood of patients with anemia and the Detection of γ globin and fetal hemoglobin expression

The genetically modified hematopoietic stem cells of Example 5 are subjected to erythroid differentiation *in vitro* as described in Example 4-1. Differentiation results are shown in FIG.23. The results of the experiments show that, the erythroid differentiation efficiencies of the control group, Enhancer-2, Enhancer-3, Enhancer-4, Enhancer-5 and Enhancer-6 are similar, and the cell membrane proteins CD71 and CD235a are highly expressed.

12 days later, mRNA of cells after erythroid differentiation is extracted and reverse-transcribed into cDNA. The expressions of BCL11A, HBB, HBG, HBA and other genes are determined by quantitative fluorescence PCR, as shown in FIG. 24. The results show that Enhancer-2, Enhancer-3, Enhancer-4 and Enhancer-5, but not Enhancer-6, down-regulate the expression of BCL11A gene, wherein Enhancer-2 has the most significant effect that the expression is down-regulated by about 1-fold. In addition, each of Enhancer-2, Enhancer-3, Enhancer-4, Enhancer-5 and Enhancer-6 increases the expression of γ globin, wherein Enhancer-2 has the most significant effect that the expression of γ globin is enhanced by 9-fold, meeting the standard of clinical treatment. Compared with the results in the existing literatures, firstly, the hematopoietic stem cells derived from severe thalassemia patients are used as the original cells for the accurate evaluation of the method according to the invention, and the method is verified to meet the requirements of clinical treatment, while in the existing literatures, only normal human bone marrow or peripheral blood samples are used; secondly, the increased folds of fetal hemoglobin expression reported in the prior literatures are only 4-7 folds, which is less than the increased proportion of fetal hemoglobin expression in the present invention. Thus, it shows that the experimental results of the present invention are significantly superior to that in the existing literatures reports (Chang et al. Methods & Clinical Development. 2017; mattew C et al. Nature.2015).

In addition, in order to more accurately evaluate the effects on the protein expression levels of fetal hemoglobin (HbF) and normal hemoglobin (HbA) caused by the gene editing of BCL11A enhancer locus with Enhancer-2, Enhancer-3, Enhancer-4, Enhancer-5 and Enhancer-6, in the present invention, we extract the proteins of the erythrocytes obtained 12 days after differentiation of the hematopoietic stem cells, performing HPLC assay (High Performance Liquid Chromatography), as shown in FIGs. 27, 28, and 29. The results show that, 1) the chromatographic peaks with the retention times of about 5.4 min and 10 min represent HbF and HbA, respectively; 2) as compared with cells not being genetically modified, cells genetically modified with Enhancer-2, Enhancer-3, Enhancer-4, Enhancer-5, and Enhancer-6 genes have higher HbF expression (higher peak and larger peak area) and lower HbA expression (lower peak and smaller peak area), indicating that the gene editing pf BCL11A Enhancer locus increases HbF expression and down regulates HbA expression; 3) among them, the ratio of HbF/HbA in cells of Mock group is about 0.79, while the ratio of HbF/HbA in cells of Enhancer-2 group is about 5.28, which is increased by 6.68 times and significantly higher than the ratio of HbF/HbA in the cells of the Enhancer-3, Enhancer-4, Enhancer-5, and Enhancer-6 group. Clinically, as for patients with severe β thalassemia as described in the context of the present invention, the hemoglobin in the blood consists of a small amount of HbA and a majority of HbF, due to the absence of β globin. Typically, there's about 20 g/L hemoglobin in patients with severe β thalassemia, wherein the proportion of HbF is up to 90% or more. Accordingly, taking the proportion of 90% as an example, the expression of HbF in genetically modified cells increases 6.68 times, the final hemoglobin content is 20×0.9×6.68+20×0.1 = 122.24 g/L, which absolutely meets the standard of clinical treatment, as the hemoglobin expression in normal subjects is 115-150 g/L (Antonio Cao, et al. Genes in Medicine. 2010; guidelines for Diagnosis and Treatment of Severe B thalassemia in 2010). Compared with the prior literatures, the invention is the only research in which the effect of gene editing of BCL11A enhancer in the hematopoietic stem cells of β thalassemia patients on improving the fetal hemoglobin expression is accurately evaluated by HPLC experiment, and the result shows that the expression of the fetal hemoglobin is significantly enhanced and meets the requirements for the clinical treatment of severe β thalassemia patients (Chang et al. Methods & Clinical Development. 2017; mattew C et al. Nature.2015; lin Ye, et al. PNAS. 2016; matthew H. Porteus, Advances in Experimental Medicine and Biology. 2013).

### Example 7: In vitro Colony-formation of genetically modified hematopoietic stem cells derived from peripheral blood of anemia patients

The hematopoietic stem cells derived from peripheral blood of anemia patients are transfected with Cas9 mRNA and Enhancer-2 by electroporation under the electroporation conditions of 300 V, 1 ms. 500-800 cells are resuspended in 1 ml of the mixture of H4434 (available from STEM CELLS TECHNOLOGY, Canada), IMDM (available from Thermo Fisher) and FBS (available from Thermo Fisher). The number of the formed colonies with different morphologies such as CFU-M, BFU-E, CFU-E, CFU-G, CFU-GM, and GEMM are observed under microscope 14 days later. The results are shown in FIG. 25.

The experimental data show that, compared with the hematopoietic stem cells not being genetically modified, the *in vitro* differentiation function of the genetically modified cells is normal, and the cells are differentiated into colonies of different lineages in blood system.

### Example 8: Off-target effect of the genetically modified hematopoietic stem cells derived from peripheral blood of anemia patients

The experiment relates to a gene sequencing method, in particular to an off-target effect analysis of the genetically modified hematopoietic stem cells derived from peripheral blood of an anemia patient by the next generation sequencing (NGS) technology.

The hematopoietic stem cells from peripheral blood of anemia patients are transfected with Cas9 mRNA and Enhancer-2 by electroporation under the electroporation conditions of 300 V, 1 ms. After 4 days of expansion, the genome of the cells is extracted and analyzed by next generation sequencing. 14 potential off-target sites are predicted by software, and the results are shown in FIG. 22.

The experimental data shows that, the on-target efficiency of gene editing is 76%, and the proportion of the 14 potential off-target sites is less than 0.3%, which is equivalent to the error of the next generation sequencing per se. Therefore, in the range of the sequencing error, off-target phenomenon caused by gene editing is not detected. Thus, this gene editing scheme is safe.

As shown in the data of the above examples, as for cells derived from different sources, Enhancer-2 shows the best effect.

### Example 9

The peripheral blood-derived hematopoietic stem cells of an anemia patient are transfected with Cas9 mRNA and Enhancer-2 by electroporation under the electroporation conditions of 300v, 1ms; followed by erythroid differentiation through the "two-step method" of the above Example 4; except in Example 9, amplifying the cells for 4 days, then differentiating them for 14 days. After 14 days, HbF staining is performed, and cells with high expression of HbF (about 10%) and low expression of HbF (about 10%) are obtained by flow cytometry sorting, and the genomes are extracted separately for the next generation sequencing analysis (deep sequencing analysis.

The steps of HbF staining are as follows: 1) collecting the cells in the culture plate, centrifuging at 600 g for 5 min, then removing the supernatant; 2) adding phosphate buffer containing 0.05% glutaraldehyde precooled at 4°C, and fixing at room temperature for 10 minutes, then centrifuging at 600 g for 5 min and removing the supernatant; 3) adding phosphate buffer containing 0.1% BSA, resuspending by pipetting, centrifuging at 600 g for 5 min and removing the supernatant; repeating three times to remove residual glutaraldehyde as much as possible; 4) adding 0.1% BSA phosphate buffer containing 0.1% Triton X-100, standing at room temperature for 4 min, centrifuging at 600 g for 5 min and removing the supernatant; 5) adding phosphate buffer containing 0.1% BSA, resuspending by pipetting, centrifuging at 600g for 5min and removing the supernatant; 6) adding 100 µl of phosphate buffer containing 0.1% BSA, adding 5 µl of HbF flow cytometry antibodies, staining at room temperature for 15 minutes; 7) adding phosphate buffer containing 0.1% BSA, centrifuging at 600 g for 5 min and removing the supernatant; 8) adding 100 µl of phosphate buffer containing 0.1% BSA, resuspending by pipetting, then performing flow cytometry analysis on the computer.

After comparing the two groups of cells with high expression of HbF and low expression of HbF, the results of the analysis are shown in Fig. 31. According to Fig. 31, it can be seen that there are mainly five types of genotypes in the cell population with high expression of HbF, wherein the efficiency of the wild type is 46.47%, the proportion of "-5" is 14.38%, the proportion of "-1" is 14.60%, the proportion of "-4" is 14.55%, and the proportion of "+1" is 10.00%. The results indicate that cells with high expression of fetal hemoglobin HbF are mainly the four genotypes of "-5", "-1", "-4", and "+1". It can be seen from the above four genotypes that, the sequence "CTTCCT" is a key target sequence (the positions pointed by the arrows in Fig. 31). The results according to Example 9 show that, if the sequence is disrupted in the genetically modified cells, the expression of fetal hemoglobin may be effectively up-regulated.

According to the results of the genetic analysis, the base sequence of "CTTCCT" (6 bp) comprises the binding sites of the STAT1 and ELF1 genes. Among them, according to the literatures, the binding site of STAT1 is the "TTCCnGGA" motif (n stands for any base) (Raja, et al. Nucleic Acids Research. 2008; George, et al. Journal of Biological Chemistry. 2001; Christoph, et al. Plos one. 2010), while the binding site of ELF-1 is the "TTCC" motif (Steven, et al. Scientific Reports. 2015; Yuang-Taung Juang, et al. The Journal of Immunology. 2007).

Among them, STAT1 and ELF1 are key regulatory transcription factors involved in blood system development and erythrocyte development, wherein STAT1 forms a regulatory network by binding to the transcription factor BCL11A, negatively regulating erythrocyte development (Jason D Buenrostro, et al. BioRxiv.2017; Keita Kirito, et al. Blood. 1998); while in the development of the blood system, the transcription factor ELF-1 forms a complex with GATA-2, and FLI-1, activating the expression of the downstream key transcription factor GATA1 (Gemma, et al. Developmental Biology. 2006).

The experimental results suggest that, the disruption of the binding sites of STAT1 and ELF-1 by disrupting the 6-bp base sequence of "CTTCCT" in the BCL11A gene prevents STAT1 and ELF-1 from binding to the BCL11A enhancer, eliminating the inhibition of fetal hemoglobin synthesis by the BCL11A gene, thereby increasing the expression of fetal hemoglobin in the blood.

### INDUSTRIAL APPLICABILITY

According to the invention, the method provided herein has the following advantages: firstly, the method may be used for gene editing of the hematopoietic stem cells derived from thalassemia patients, and it completely meets the requirements for the clinical treatment of thalassemia and sickle cell anemia; secondly, by using the chemically modified sgRNA, the gene editing efficiency of the method is high, the expression of fetal hemoglobin is remarkably improved, and the cells can be used for repopulating the hematopoietic system of a mouse model; and finally, the off-target analysis shows a high degree of safety. Accordingly, the method developed in the present invention has the potential to replace the conventional hematopoietic stem cell transplantation therapy technique for curing patients with severe thalassemia and sickle cell anemia.

## Claims

1. An *in vitro* method for increasing the expression of fetal hemoglobin (HbF) in human hematopoietic stem cells, comprising:
disrupting a BCL11A genomic region from 0-15 nucleotides upstream of the CTTCCT sequence to 0-15 nucleotides downstream of the CTTCCT sequence in the chromosome 2 in the hematopoietic stem cells by gene editing technology, wherein the BCL11A genomic region is located in a region from chr2:60495236 to chr2:60495271,
wherein the gene editing technology is a CRISPR/Cas9 gene editing technology, comprising introducing an sgRNA comprising a sequence selected from any one of SEQ ID NOs: 3, 4, 8 and 33-63 into the hematopoietic stem cells to edit the BCL11A genomic region, and
wherein the sgRNA and a Cas9-encoding mRNA are co-introduced into the hematopoietic stem cells by electroporation.

2. The *in vitro* method according to claim 1, wherein the sgRNA is 2'-O-methyl modified and/or internucleotide 3'-thio modified, in particular, the chemical modification is 2'-O-methyl modification of the first one, two, and/or three bases at the 5' end and/or the last base at the 3' end of the sgRNA.

3. The *in vitro* method according to claim 2, wherein the electroporation conditions are 200-600 V, 0.5 ms-2 ms.

4. A method for preparing mature erythrocytes or precursor cells thereof being genetically modified to increase the expression of fetal hemoglobin (HbF), which comprises:
(a) obtaining genetically modified hematopoietic stem cells by the method of any one of claims 1-3;
(b) performing hematopoietic stem cell erythroid expansion and differentiation of the genetically modified hematopoietic stem cells by using a HSPCs erythroid expansion and differentiation medium,
wherein the HSPCs erythroid expansion and differentiation medium comprises a basal medium, and a composition of growth factors, and wherein the composition of growth factors comprises a stem cell growth factor (SCF), interleukin 3 (IL-3) and erythropoietin (EPO).

5. The method according to claim 4, further comprising:
performing hematopoietic stem cell erythroid differentiation and enucleation by using an erythroid differentiation and enucleation medium,
wherein the erythroid differentiation and enucleation medium comprises a basal medium, growth factors, and antagonists and/or inhibitors of a progesterone receptor and a glucocorticoid receptor.

6. The method according to claim 5, wherein the growth factors in the erythroid differentiation and enucleation medium comprise erythropoietin (EPO), and the antagonists and/or inhibitors of a progesterone receptor and a glucocorticoid receptor are any one, or two or more selected from the following compounds (I)-(IV):

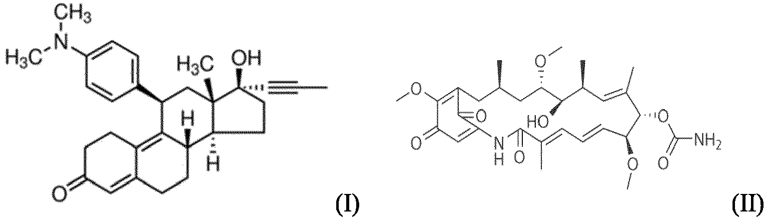

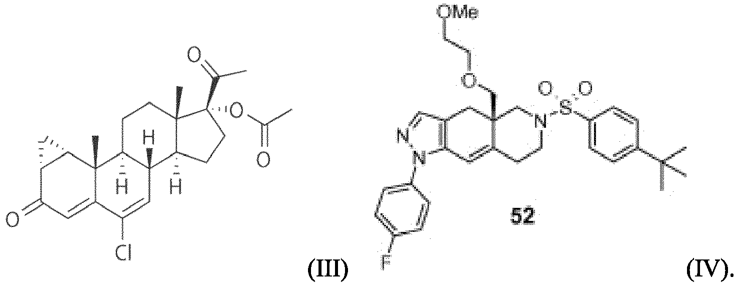

## Patentansprüche

1. *In-vitro-Verfahren* zur Erhöhung der Expression von fetalem Hämoglobin (HbF) in menschlichen hämatopoetischen Stammzellen, umfassend:
das Unterbrechen einer genomischen BCL11A-Region von 0-15 Nukleotiden stromaufwärts der CTTCCT-Sequenz bis 0-15 Nukleotiden stromabwärts der CTTCCT-Sequenz im Chromosom 2 in den hämatopoetischen Stammzellen durch Gen-Editierungstechnologie, wobei sich die genomische BCL11A-Region in einer Region von chr2:60495236 bis chr2:60495271 befindet,
wobei die Gen-Editierungstechnologie eine CRISPR/Cas9-Gen-Editierungstechnologie ist, die das Einbringen einer sgRNA mit einer Sequenz, die aus einer der SEQ ID NOs: 3, 4, 8 und 33-63 ausgewählt ist, in die hämatopoetischen Stammzellen umfasst, um die genomische BCL11A-Region zu verändern, und
wobei die sgRNA und eine Cas9-kodierende mRNA durch Elektroporation gemeinsam in die hämatopoetischen Stammzellen eingebracht werden.

2. *In vitro-Verfahren* nach Anspruch 1, wobei die sgRNA 2'-O-Methyl-modifiziert und/oder Internukleotid-3'-thio-modifiziert ist, insbesondere die chemische Modifikation eine 2'-O-Methyl-Modifikation der ersten ein, zwei und/oder drei Basen am 5'-Ende und/oder der letzten Base am 3'-Ende der sgRNA ist.

3. *In-vitro-Verfahren* nach Anspruch 2, wobei die Elektroporationsbedingungen 200-600 V und 0,5 ms-2 ms betragen.

4. Verfahren zum Herstellen von reifen Erythrozyten oder deren Vorläuferzellen, die genetisch modifiziert sind, um die Expression von fetalem Hämoglobin (HbF) zu erhöhen, umfassend:
(a) das Gewinnen von genetisch modifizierten hämatopoetischen Stammzellen durch das Verfahren nach einem der Ansprüche 1 bis 3;
(b) das Durchführen einer erythroiden Expansion und Differenzierung der genetisch modifizierten hämatopoetischen Stammzellen unter Verwendung eines erythroiden Expansions- und Differenzierungsmediums für HSPCs,
wobei das erythroide Expansions- und Differenzierungsmedium für HSPCs ein Basalmedium und eine Zusammensetzung von Wachstumsfaktoren umfasst, und wobei die Zusammensetzung von Wachstumsfaktoren einen Stammzellwachstumsfaktor (SCF), Interleukin 3 (IL-3) und Erythropoietin (EPO) umfasst.

5. Verfahren nach Anspruch 4, ferner umfassend:
das Durchführen der erythroiden Differenzierung und Enukleation von hämatopoetischen Stammzellen unter Verwendung eines erythroiden Differenzierungs- und Enukleationsmediums,
wobei das erythroide Differenzierungs- und Enukleationsmedium ein Basalmedium, Wachstumsfaktoren und Antagonisten und/oder Inhibitoren eines Progesteronrezeptors und eines Glucocorticoidrezeptors umfasst.

6. Verfahren nach Anspruch 5, wobei die Wachstumsfaktoren in dem erythroiden Differenzierungs- und Enukleationsmedium Erythropoietin (EPO) umfassen und die Antagonisten und/oder Inhibitoren eines Progesteronrezeptors und eines Glucocorticoidrezeptors eine oder zwei oder mehr der folgenden Verbindungen (I)-(IV) sind:

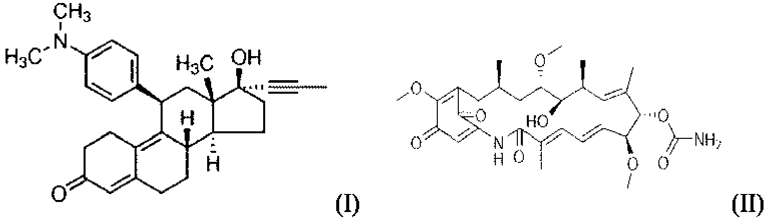

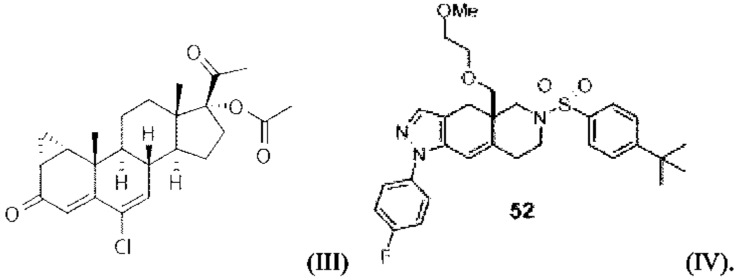

## Revendications

1. Procédé *in vitro* d'augmentation de l'expression de l'hémoglobine foetale (HbF) dans les cellules souches hématopoïétiques humaines, comprenant :
l'interruption d'une région du génome d'une BCL11A de 0 à 15 nucléotides en amont de la séquence CTTCCT à 0 à 15 nucléotides en aval de la séquence CTTCCT dans le chromosome 2 des cellules souches hématopoïétiques par une technologie d'édition génique, dans lequel la région du génome de la BCL11A est située dans une région allant de chr2:60495236 à chr2:60495271,
dans lequel la technologie d'édition génique est une technologie d'édition génique CRISPR/Cas9, comprenant l'introduction d'un ARNsg comprenant une séquence sélectionnée parmi l'une quelconque des SEQ ID N° : 3, 4, 8 et 33 à 63 dans les cellules souches hématopoïétiques pour éditer la région du génome de la BCL11A, et
dans lequel l'ARNsg et un ARNm codant pour Cas9 sont co-introduits dans les cellules souches hématopoïétiques par électroporation.

2. Procédé *in vitro* selon la revendication 1, dans lequel l'ARNsg est modifié par 2'-O-méthyle et/ou modifié par internucléotide 3'-thio, en particulier, la modification chimique est la modification par 2'-O-méthyle de la première, des deux et/ou des trois premières bases à l'extrémité 5' et/ou de la dernière base à l'extrémité 3' de l'ARNsg.

3. Procédé *in vitro* selon la revendication 2, dans lequel les conditions d'électroporation sont de 200 à 600 V, de 0,5 ms à 2 ms.

4. Procédé de préparation d'érythrocytes matures ou de leurs cellules précurseurs génétiquement modifiées de ceux-ci pour augmenter l'expression de l'hémoglobine foetale (HbF), qui comprend :
(a) l'obtention de cellules souches hématopoïétiques génétiquement modifiées par le procédé selon l'une quelconque des revendications 1 à 3 ;
(b) la réalisation de l'expansion et de la différenciation érythroïdes des cellules souches hématopoïétiques génétiquement modifiées en utilisant un milieu d'expansion et de différenciation érythroïde des CSPH,
dans lequel le milieu d'expansion et de différenciation érythroïdes des CSPH comprend un milieu de base et une composition de facteurs de croissance, et dans lequel la composition de facteurs de croissance comprend un facteur de croissance des cellules souches (SCF), l'interleukine 3 (IL-3) et de l'érythropoïétine (EPO).

5. Procédé selon la revendication 4, comprenant en outre :
la réalisation de la différenciation et de l'énucléation érythroïdes des cellules souches hématopoïétiques en utilisant un milieu de différenciation et d'énucléation érythroïdes,
dans lequel le milieu de différenciation et d'énucléation érythroïdes comprend un milieu basal, des facteurs de croissance et des antagonistes et/ou des inhibiteurs d'un récepteur de la progestérone et d'un récepteur des glucocorticoïdes.

6. Procédé selon la revendication 5, dans lequel les facteurs de croissance dans le milieu de différenciation et d'énucléation érythroïdes comprennent l'érythropoïétine (EPO), et les antagonistes et/ou inhibiteurs d'un récepteur de la progestérone et d'un récepteur des glucocorticoïdes sont un composé ou deux ou plus quelconques choisis parmi les composés (1) à (IV) suivants :
